# EUROPEAN PATENT SPECIFICATION

(11) **EP 4 414 460 B1**
(45) Date of publication and mention of the grant of the patent: **29.04.2026**
(21) Application number: 23155436.1
(22) Date of filing: 07.02.2023
(51) Int. Cl.: C12Q 1/6869

(54) **COMPOSITIONS COMPRISING A SEQUENCE SPECIFIC ENDORIBONUCLEASE AND METHODS OF USE**
ZUSAMMENSETZUNGEN MIT EINER SEQUENZSPEZIFISCHEN ENDORIBONUKLEASE UND VERFAHREN ZUR VERWENDUNG
COMPOSITIONS COMPRENANT UNE ENDORIBONUCLÉASE SPÉCIFIQUE D'UNE SÉQUENCE ET PROCÉDÉS D'UTILISATION

(43) Date of publication of application: 14.08.2024
(73) Proprietor: Arcticzymes AS, 9091 Tromsø (NO)
(72) Inventor: KETELSEN STRIBERNY, Bernd, N-9103 KVALØYA (NO); ROTHWEILER, Ulli, N-9024 Tromsø (NO)
(74) Representative: Acapo Onsagers AS

(56) References cited:
- WO-A1-2017/098468
- US-A1- 2019 309 337
- MANIKANDAN PARTHASARATHY ET AL: "Identification, functional characterization, assembly and structure of ToxIN type III toxin-antitoxin complex from E. coli", NUCLEIC ACIDS RESEARCH, vol. 50, no. 3, 22 February 2022 (2022-02-22), GB, pages 1687 - 1700, XP093189859, ISSN: 0305-1048, DOI: 10.1093/nar/gkab1264
- MANIKANDAN PARTHASARATHY ET AL: "Supporting Information for: Identification, functional characterization, assembly and structure of ToxIN type III toxin-antitoxin complex from E. coli", NUCLEIC ACIDS RESEARCH, 8 January 2022 (2022-01-08), pages 1 - 10, XP093274426, Retrieved from the Internet <URL:https://academic.oup.com/nar/article/50/3/1687/6501237#supplementary-data> DOI: 10.1093/nar/gkab1264
- GUEGLER CHANTAL K. ET AL: "Shutoff of host transcription triggers a toxin-antitoxin system to cleave phage RNA and abort infection", MOLECULAR CELL, vol. 81, no. 11, 3 June 2021 (2021-06-03), AMSTERDAM, NL, pages 2361 - 2373.e9, XP093058945, ISSN: 1097-2765, DOI: 10.1016/j.molcel.2021.03.027

## Description

### FIELD OF THE INVENTION

The present disclosure provides compositions comprising sequence specific endoribonucleases and methods of their use in RNA analysis and RNA synthesis. In particular the present disclosure relates to compositions comprising ToxN endoribonucleases. ToxN endoribonucleases recognises and cleaves single stranded ribonucleic acid (RNA) molecules.

### INTRODUCTION

Endoribonucleases are a group of enzymes that cleaves internal phosphodiester bonds between adjacent nucleotides of RNA in either single-stranded RNAs or double-stranded RNAs depending on the enzyme. The endoribonuclease may be sequence specific (e.g. restriction endoribonucleases) or sequence independent.

A number of endoribonuclease enzymes are known, such as for example RNase H which is a family of sequence independent endoribonucleases that catalyse the cleavage of RNA in an RNA/DNA hybrid substrate.

Endoribonuclease enzymes have several applications within molecular biology research. For instance, removal of RNA in DNA extraction processes and recombinant protein purifications, cDNA synthesis, RNA fingerprinting, and detection of RNA modifications, e.g. 5'capping of mRNA.

Therapeutic RNA molecules such as mRNA molecules encoding antigens for vaccine production, represent an emerging class of drugs. Successful protein expression from transfected mRNA depends in addition to transfection efficiency also on mRNA stability and translation efficiency. 5'cap structure and 3' poly(A) tail are important features for obtaining high translation efficiency. Efficient methods for determining 5'capping efficiency of mRNA or other RNA modifications is therefore highly desirable.

Analytical methods such as gel electrophoresis, ion-pair reverse-phase high-performance liquid chromatography (IP RP HPLC) or mass spectrometry (MS) are commonly used for analysing RNA modifications.

However, identification of capping completeness of long RNA molecules, i.e. RNA molecules longer than 5-10 ribonucleotides is problematic because capping results in only a small shift in molecular weight of approximately 600 Da, which equals roughly to one ribonucleotide. Such small shift in molecular weight prevents a direct down-stream gel-based or mass spectrometry (MS) analysis of long mRNA molecules due to poor resolution.

Alternative capping molecules result in different products exhibiting only small shifts in molecular weight: e.g. Cap-1 or Cap-0, 5'-triphosphate, 5'-diphosphate, unmethylated G-cap, reverse cap.

In order to overcome the problem with poor resolution of existing current analytical tools, RNA samples and in particular samples comprising long RNA molecules need to be cleaved into shorter fragments before further analysis.

Today's standards for cleavage of RNA are endoribonuclease-based cleavage with either enzymes with high cutting frequency, such as RNase I, RNase H-based methods, ribozyme-based methods, or DNAzyme-based approaches.

Endoribonucleases with high cutting frequency like RNase I cleaves single stranded RNA after each G. Such enzymes are not optimal for certain RNA analysis methods since they lead to a high degree of fragmentation.

RNase H which cleaves a RNA/DNA hybrid is dependent on specific DNA-hybridization, AU2016297778, and RNase H-based methods have problems with unspecific and incomplete cleavage of target RNA even if the DNA probe is correctly hybridized. Therefor RNase H-based methods requires optimalisation of conditions for each hybridized RNA-DNA oligonucleotide pair in order to achieve complete and specific digestion of the RNA.

The Csy4 endoribonuclease is dependent on a guide RNA for recognition and cleavage of its RNA target sequence.

Just recently, ribozymes have been described to be applicable for sequence-specific cleavage of mRNA as well, Vlatkovic et al., Ribozyme assays for quantifying the capping efficiency of in vitro transcribed mRNA, Pharmaceutics 2022, vol. 14, no.2, p.328, and WO2015101416.

However, ribozymes rely of synthesis of ribonucleic acids and need to be used in 1-10-fold excess over the concentration of the substrate to be analysed. Ribozymes are catalytic RNA molecules and are thus more expensive to produce and also more challenging to work with due to lack of stability.

DNAzymes are DNA oligonucleotides with catalytic activity, similar to ribozymes. The most abundant class of deoxyribozymes are ribonucleases which catalyse cleavage of a ribonucleotide phosphodiester bond, Hengesbach, M. et al. Use of DNAzymes for site-specific analysis of ribonucleotide modification, RNA 2008, vol.14, no.1, p.180-187.

Guegler et al 2021, "Shut off of host transcription triggers toxin-antitoxin system to cleave phage RNA and abort infection", Molecular cell vol 81, no. 11, mentioned that Toxin-antitoxin (TA) systems are widespread in bacteria, but their activation mechanisms and *bona-fide* targets remain largely unknown. Their results reveal a critical trade-off in blocking host transcription: it helps phage commandeer host resources, but can activate potent defense systems. More generally, the results now reveal the native targets of an RNase toxin and activation mechanism of a phage-defensive TA system.

WO2017098468A1 suggests a method that provides those of skill in the biotechnological arts with a way of identifying the 5' cap without the need for radiolabels, by using liquid chromatography coupled to electrospray mass spectrometry (LC-MS) and detecting their differences in mass and retention time. Using mass spectrometry as the detector allows identification based on an intrinsic property of the species that can be measured accurately and with high-resolution. Unambiguous identification of 5' cap cleavage products by mass spectrometry also enables identification of a variety of uncapped species.

US2019309337A1 claimed a T7 ribonucleic acid (RNA) polymerase variant modified to comprise an amino acid substitution at position G47, wherein the amino acid substitution causes a loop structure of the T7 RNA polymerase variant to undergo a conformational change to a helix structure as the T7 RNA polymerase variant transitions from an initiation complex to an elongation complex.

Despite the existence of endoribonucleases there is a continued need for providing further endoribonucleases which permit efficient and simplified methods for RNA analysis or RNA synthesis that overcome one or more of the disadvantages of the endoribonucleases and the methods of prior art.

The inventors have surprisingly and for the first time shown that a sequence specific endoribonuclease ToxN from Type III toxin-antitoxin systems cleaves single stranded RNA specifically at its recognition site in the presence of particular concentrations of a monovalent salt, i.e. unspecific catalytic activity (also called star-activity") of the enzyme is reduced in the absence or at low to moderate concentrations of monovalent salt.

The inventors have also for the first time determined that, surprisingly, the catalytic activity of ToxN is inhibited by certain concentrations of divalent metal cations, which is in contrast to other endoribonucleases, i.e. the enzyme tolerate certain low concentrations of a divalent metal cation, but its enzyme activity is inhibited at higher concentrations.

The inventors have also for the first time determined that, surprisingly, the unspecific catalytic, i.e. its star-activity of ToxN from E. coli is reduced or absent at low concentrations of a divalent metal cation. The family of ToxN enzymes are thus not dependent on divalent metal cations for their catalytic activity.

The inventors have also shown that it is not necessary to remove divalent metal cations from a reaction mixture comprising a ToxN, rather the sequence specific catalytic activity may be obtained in the presence of a divalent metal cation chelator such as EDTA or EGTA. This is a great advantage as RNA samples are often stored in a buffer comprising EDTA to prevent degradation from RNases.

Such sequence specific catalytic activity at certain concentrations of monovalent salts and reduced unspecific cleavage of RNA in the absence or at low concentrations of divalent metal cations are not observed with other well-known endoribonucleases.

Divalent metal cations are known to stabilise RNA and protein three dimensional structures.

Without being bond by the theory, cleavage of RNA in the absence or at low concentrations of divalent metal cations may be more complete as such reaction conditions destabilizes the RNA three dimensional structures thereby improving the ToxN's accessibility to its target site in the RNA molecule thereby decreasing the amount of enzyme needed for a complete digestion.

Further, as mentioned above, this class of endoribonucleases have the advantage that they digest single stranded RNA at specific sites without the need of a hybridized DNA probe or an RNA guide oligo. The family of ToxN enzyme is also more efficient and more stable compared to catalytic nucleic acids such as ribozymes or DNAzymes which requires 1-10-fold excess over the concentration of the RNA substrate.

The above-mentioned advantages of the ToxN endoribonucleases compared to other well know endoribonucleases makes this family of endoribonucleases particularly useful in *in vitro* methods for analysing modification of RNA molecules such as efficiency of 5'capping and poly(A)-tail generation of synthetically transcribed RNA molecules. There is also provided herein the use of ToxN in methods for RNA fingerprinting and in methods for producing precursor RNA molecules from rolling circle transcription (RCT).

### SUMMARY OF THE INVENTION

The protected invention is defined by the claims. The present invention concerns a method of cleaving single stranded RNA molecules in a sample, wherein the method comprises the steps:
a. providing a sample comprising at least one single stranded RNA molecule comprising a cleavage site for a ToxN endoribonuclease, wherein said single stranded RNA molecule is a concatemer comprising multiple copies of precursors of either mRNA, siRNA, circular RNA, microRNA or ribozyme and wherein the concatemeric RNA molecule comprises a cleavage site for a ToxN endoribonuclease between each copy of precursor of mRNA, siRNA, circular RNA, microRNA or ribozyme; and
b. contacting a ToxN endoribonuclease or an enzymatically active fragment thereof with the at least one RNA molecule in said sample under conditions which permits cleavage of at least a portion said RNA molecule present in the sample and wherein concentration of a monovalent salt in the sample is ≤150 mM, and wherein the monovalent salt is an alkali metal salt.

The present invention concerns a method for determining 5'-capping efficiency of a RNA molecule wherein the method comprises the steps:
a. providing a sample comprising at least one single stranded mRNA molecule comprising a cleavage site for a ToxN endoribonuclease in the 5'UTR of said mRNA;
b. contacting said sample from step a) with a ToxN endoribonuclease under conditions that permits cleavage of at least a portion of said single stranded RNA molecules to produce at least one 5' terminal RNA fragment and at least one 3' terminal RNA fragment, wherein concentration of a monovalent salt in the sample is ≤150 mM, and wherein the monovalent salt is an alkali metal salt; and
c. separating and detecting the RNA fragments from step b) and determine the presence of a 5'-capping modification at the 5'end of said 5' terminal RNA fragments.

The present invention concerns a method of RNA fingerprinting, wherein the method comprises the steps:
a. providing a sample comprising a at least one single stranded RNA molecule with unknown sequence;
b. contacting said sample from step a) with a ToxN endoribonuclease under conditions that permit cleavage of at least a portion of said RNA molecules thereby obtaining a plurality of RNA fragments, wherein concentration of a monovalent salt in the sample is ≤150 mM, and wherein the monovalent salt is preferably an alkali metal salt; and
c. separation and detection of the fragmented RNA molecule from step b, thereby obtaining a fingerprint of said RNA molecule with unknown sequence and compare the obtained fingerprint with fingerprints from RNA molecules with known sequence.

In one aspect, the separation and detection step is selected from gel electrophoresis, capillary gel-electrophoresis (CGE), PAGE, high pressure liquid chromatography (HPLC), mass spectrometry (MS) or LC-MS, IP RP HPLC and LC-UV.

In one aspect, the RNA molecule is selected from mRNA, RNA virus, an immunogenic RNA molecule, viroid, long non-coding RNA and ribozyme.

In one aspect, the sample is essentially without Mg²⁺ or Mn²⁺.

In one aspect, a concentration of divalent metal cations in the sample is ≤ 3mM, wherein the divalent metal cations are Mg²⁺ or Mn²⁺.

In one aspect, the sample comprises a concentration of a divalent ion chelating agent of ≤ 10mM.

In one aspect, the isolated ToxN endoribonuclease or an enzymatically fragment thereof comprises amino acid sequence of SEQ ID No.1 or comprising an amino acid sequence which is at least 70% identical to SEQ ID No.1.

However, the present disclosure also describes a composition comprising isolated ToxN endoribonuclease or an enzymatic fragment thereof, wherein a concentration of a monovalent salts in the composition is ≤150 mM, such as about ≤100 mM and wherein the monovalent salt is preferably an alkali metal salt.

The composition can comprise a concentration of an alkali metal salt about ≤75 mM, such as about ≤55 mM, such as from about 20 mM to about 75 mM, such as from about 20 mM to about 55 mM.

The composition can be a solution for application to a sample comprising at least one polyribonucleic acid (RNA) molecule.

The sample can have a volume from about ≥ 0.1µl.

The sample can have a volume from 0.1µl about to about 500µl, preferably from about 0.1 µl to about 300 µl, preferably from about 0.1 µl to about 250 µl, preferably from about 0.1 µl to about 200 µl, more preferably from about 0.1 µl to about 150 µl, more preferably from about 0.1 µl to about 100 µl, more preferably from about 0.1 µl to about 75 µl, more preferably from about 0.1 µl to about 50 µl.

The monovalent salt of the composition or sample can be an inorganic salt comprising alkali metal ions.

Thus, the monovalent salt is preferably an alkali metal salt.

The alkali metal ions of the salt can be selected from Na+, K+, Li+, Rb+, Cs+ and Fr+ or any combinations thereof.

The alkali metal ions can be selected from Na+, K+, Li+ and Rb+.

The salts comprising alkali metals ions can be preferably selected from fluorine (F), chlorine (Cl), bromine (Br), iodine (I), sulphates, phosphates or hydroxides or any suitable combinations thereof.

The alkali metal salt can be selected from NaCl, KCl, Na2SO4, K2SO4, KOH, NaOH, Na-Phosphates, K-Phopshates or any suitable combinations.

The composition can be essentially without divalent metal cations.

The divalent metal cations are preferably Mg²⁺ or Mn²⁺.

The composition can be essentially without divalent metal cations, i.e. a concentration of divalent metal cations in the composition is about ≤ 3mM, preferably about ≤ 2mM, more preferably about ≤ 1mM.

The composition can be essentially without divalent metal cations, i.e. comprises ratio of concentration of a divalent metal cation to the concentration of divalent ion chelating agent in the composition providing that the concentration of free divalent metal cation present in the composition is about ≤3 mM, preferably about ≤ 2mM, more preferably about ≤ 1mM.

The composition can comprise a concentration of a divalent ion chelating agent of about ≤ 10mM.

The divalent ion chelator is preferably EDTA or EGTA.

The isolated ToxN enzyme can be a ToxN enzyme from E. coli.

The isolated ToxN endoribonuclease or an enzymatically fragment thereof can comprise amino acid sequence of SEQ ID No.1 or comprising an amino acid sequence which is at least 70% identical to SEQ ID No.1.

The isolated ToxN endoribonuclease or an enzymatically fragment thereof can comprise an amino acid sequence of SEQ ID No.1 or comprises an amino acid sequence which is at least 75%, 80%, 85%, 90%, 92%, 94%, 95%, 98% or 99% identical to SEQ ID No.1.

The isolated ToxN endoribonuclease or an enzymatically fragment thereof can comprise amino acid sequence of endoribonuclease having an amino acid sequence selected from:
- SEQ ID No. 6 or an amino acid sequence which is at least 70% identical thereto,
- SEQ ID No. 7 or an amino acid sequence which is at least 70% identical thereto,
- SEQ ID No. 8 or an amino acid sequence which is at least 70% identical thereto, or
- SEQ ID No. 9 or an amino acid sequence which is at least 70% identical thereto.

The ToxN endoribonuclease can have an amino acid sequence which is at least 75%, preferably at least 80%, 85%, 90% or 95%, e.g. at least 98% or 99% or 99.5%, identical to SEQ ID Nos. 6, 7, 8 or 9.

The ToxN endoribonuclease can consist of an amino acid sequence selected from the group consisting of SEQ ID Nos.1, 6, 7, 8 and 9. Enzymatically active fragments thereof are also provided.

The isolated ToxN endoribonuclease is not in a complex with ToxI RNA.

The composition or sample can comprise a ToxN endoribonuclease or an enzymatically active fragment thereof, said ToxN endoribonuclease can comprise amino acid sequence of SEQ ID No.1 or comprise an amino acid sequence which is at least 70% identical to SEQ ID No.1 wherein
- concentration of monovalent salt in the composition or sample preferably about ≤100 mM, about ≤75 mM, about ≤55 mM, more preferably from about 20 mM to about 75 mM, more preferably from about 20 mM to about 55 mM.

The composition or sample can comprise a ToxN endoribonuclease or an enzymatically active fragment thereof, said ToxN endoribonuclease comprises amino acid sequence of SEQ ID No.1 or comprising an amino acid sequence which is at least 70% identical to SEQ ID No.1 wherein
- concentration of monovalent salt in the composition or sample preferably about ≤100 mM, about ≤75 mM, about ≤55 mM, more preferably from about 20 mM to about 75 mM, more preferably from about 20 mM to about 55 mM;
   and wherein
- the composition or sample is essentially without free divalent metal cations wherein the divalent metal cations are preferably Mg2+ or Mn2+ and wherein the divalent metal cations are provided as inorganic salts, such as MgCl2 or MnCl2.

The composition or sample can comprise a ToxN endoribonuclease or an enzymatically active fragment thereof, said ToxN endoribonuclease can comprise amino acid sequence of SEQ ID No.1 or comprise an amino acid sequence which is at least 70% identical to SEQ ID No.1 wherein
- concentration of monovalent salt in the composition or sample preferably about ≤100 mM, about ≤75 mM, about ≤55 mM, more preferably from about 20 mM to about 75 mM, more preferably from about 20 mM to about 55 mM;
   and wherein
- concentration of free divalent metal cation is about ≤1 mM, the divalent metal cations are preferably Mg²⁺ or Mn²⁺ and wherein the divalent metal cations are provided as inorganic salts such as MgCl₂ or MnCl₂.

The composition or sample can comprise a ToxN endoribonuclease or an enzymatically active fragment thereof, said ToxN endoribonuclease can comprise amino acid sequence of SEQ ID No.1 or comprise an amino acid sequence which is at least 70% identical to SEQ ID No.1 wherein
--concentration of monovalent salt in the composition or sample preferably about ≤100 mM, about ≤75 mM, about ≤55 mM, more preferably from about 20 mM to about 75 mM, more preferably from about 20 mM to about 55 mM;
and wherein
- the composition or sample comprises ratio of concentration of a divalent metal cation to the concentration of divalent ion chelating agent in the composition or sample such that the concentration of free divalent metal cation present in the sample or composition is about ≤1 mM and the concentration of divalent ion chelator is about ≤10 mM
- the divalent metal cations are preferably Mg²⁺ or Mn²⁺ and provided as inorganic salts such as MgCl₂ or MnCl₂ and
- the divalent ion chelating agent is preferably EDTA or EGTA.

There is also described a method of cleaving single stranded RNA molecules in a sample, wherein the method comprises the steps:
a. providing a sample comprising at least one single stranded RNA molecule comprising a cleavage site for a ToxN endoribonuclease; and
b. contacting a ToxN endoribonuclease or an enzymatically active fragment thereof with the at least one RNA molecule in said sample under conditions which permits cleavage of at least a portion said RNA molecule present in the sample, wherein concentration of a monovalent salt in the sample is about ≤150 mM, such as about ≤100 mM and wherein the monovalent salt is preferably an alkali metal salt.

Said single stranded RNA molecule in step a) can be a concatemer comprising multiple copies of precursors of either mRNA, siRNA, circular RNA precursors, microRNA or ribozyme and wherein the concatemeric RNA molecule comprising a cleavage site for a ToxN endoribonuclease between each copy of precursors of mRNA, siRNA, circular RNA, microRNA or ribozyme.

The cleavage step will typically be incubation which permits cleavage of at least a portion said RNA molecule present in the sample.

The incubation can take place at around 10°C to around 50°C, such as around 10°C to 30°C, preferably around 15°C.

The incubation time which permits cleavage of at least a portion said RNA molecule present in the sample is from about 1 minute to about 2 hours, such as from about 5 minutes to about 1.5 hours, such as from about 15 minutes to about 1 hour.

There is also described a method for preparing single stranded circular RNA molecules, wherein the method comprises the steps:
a. providing a sample comprising at least one single stranded RNA molecule wherein the RNA molecule comprises a cleavage site for a ToxN endoribonuclease;
b. contacting a ToxN endoribonuclease with the at least one single stranded RNA molecule under conditions that permit digestion of at least a portion of at least one RNA molecule present in the sample thereby producing at least one RNA molecule comprising a 3'-PO4 end and a 5'-OH end, wherein concentration of a monovalent salt in the sample is about ≤150 mM, such as about ≤100 mM and wherein the monovalent salt is preferably an alkali metal salt, and
c. contacting at least one cleaved RNA molecule with RtcB ligase under conditions which permit ligation thereby producing circular RNA.

There is also described a method of synthesizing siRNAs. The method comprising the steps:
a. providing a sample comprising at least one rolling circle transcribed concatemeric RNA molecule comprising cleavage sites for two different ToxN endoribonucleases, ToxN-A and ToxN-B, having different recognition sites, wherein the recognition sites for ToxN-B is situated between tandem repeats and recognition sequences for ToxN-A is situated within the tandem repeats;
b. contacting said sample with the ToxN-B endoribonuclease or an enzymatically active fragment thereof under conditions which permits cleavage of at least a portion of said RNA molecules present in the sample thereby producing single repeats that form a hairpin structure based on their sense-antisens sequence information; contacting the formed hairpin structures with a second ToxN-A enzyme that cleaves the RNA in the loop structure to form double stranded RNA molecules, wherein concentration of a monovalent salt in the sample is about ≤150 mM, such as about ≤100 mM and wherein the monovalent salt is preferably an alkali metal salt; and
c. the resulting overhangs containing parts of the ToxN recognition sites is removed by using a standard single-strand specific ribonuclease such as RNase T1 thereby producing double stranded siRNAs.

There is also described a method for determining 5'-capping efficiency of a RNA molecule wherein the method comprises the steps:
a. providing a sample comprising at least one single stranded mRNA molecule comprising a cleavage site for a ToxN endoribonuclease in the 5'UTR of said mRNA;
b. contacting said sample from step a) with a ToxN endoribonuclease under conditions that permits cleavage of at least a portion of said single stranded RNA molecules to produce at least one 5' terminal RNA fragment and at least one 3' terminal RNA fragment; wherein concentration of a monovalent salt in the sample is about ≤150 mM, such as about ≤100 mM and wherein the monovalent salt is preferably an alkali metal salt; and
c. separating and detecting the RNA fragments from step b) and determine the presence of a 5'-capping modification at the 5'end of said 5' terminal RNA fragments.

The 5' terminal RNA fragment can have a length from about 2 ribonucleotides to about 100 ribonucleotides, preferably 5 to 50, more preferably 5 to 10.

There is also described a method for determining Poly(A) tail length distribution of a RNA molecule wherein the method comprises the steps:
a. providing a sample comprising at least one single stranded mRNA molecule comprising a cleavage site for a ToxN endoribonuclease upstream of the Poly(A) tail of said mRNA;
b. contacting said sample from step a) with a ToxN endoribonuclease under conditions that permits cleavage of at least a portion of said single stranded RNA molecules to produce at least one 5' terminal RNA fragment and at least one 3' terminal RNA fragment, wherein concentration of a monovalent salt in the sample is about ≤150 mM, such as about ≤100 mM and wherein the monovalent salt is preferably an alkali metal salt; and
c. separating and detecting the RNA fragments from step b) and determine the length of the Poly(A) tail at the 3'end of said 3' terminal RNA fragments.

In one embodiment of the fifth aspect the composition and sample comprising ToxN is the composition and sample as described in the first aspect and embodiments thereof.

There is also described a method of RNA fingerprinting, wherein the method comprises the steps:
a. providing a sample comprising a at least one single stranded RNA molecule with unknown sequence;
b. contacting said sample from step a) with a ToxN endoribonuclease under conditions that permit cleavage of at least a portion of said RNA molecules thereby obtaining a plurality of RNA fragments, wherein concentration of a monovalent salt in the sample is about ≤150 mM, such as about ≤100 mM and wherein the monovalent salt is preferably an alkali metal salt; and
c. separation and detection of the fragmented RNA molecule from step b, thereby obtaining a fingerprint of said RNA molecule with unknown sequence and compare the obtained fingerprint with fingerprints from RNA molecules with known sequence.

In one embodiment of the sixth aspect the composition and sample comprising ToxN is the composition and sample as described in the first aspect and embodiments thereof.

There is also described a method for analysing a multivalent RNA composition, wherein the method comprises the steps:
a. providing a sample comprising a multivalent RNA composition comprising a first RNA species and a second RNA species comprising a cleavage site for a ToxN endoribonuclease in at least one position of said first RNA species and said second RNA species;
b. contacting said sample from step a) with a ToxN endoribonuclease under conditions that permits cleavage of at least a portion of said first RNA species and said second RNA species thereby releasing a plurality of first RNA fragments and second RNA fragments from the first and second RNA species, wherein concentration of a monovalent salt in the sample is about ≤150 mM, such as about ≤100 mM and wherein the monovalent salt is preferably an alkali metal salt; and
c. separation and detection the presence and/or amount of the released first RNA fragments and second RNA fragments.

The RNA molecule can be selected from mRNA, RNA virus, an immunogenic RNA molecule, viroid, long non-coding RNA and ribozyme.

The separation and detection step can be based on different molecular weight, charge or length of the RNA fragments.

The separation and detection step can be selected from gel electrophoresis, capillary gel-electrophoresis (CGE), PAGE, high pressure liquid chromatography (HPLC), mass spectrometry (MS) or LC-MS, IP RP HPLC and LC-UV.

There is also described a kit comprising:
a. The composition according to the first aspect and embodiments therein; and
b. A second composition comprising a second enzyme selecting from a group consisting of an RNA polymerase, RNA ligase for ligating single stranded RNA molecules, a pyrophosphatase, a phosphatase, a kinase or any other nucleic acid or ribonucleic acid modifying enzymes and at least one further ToxN endoribonuclease with a different recognition site from the ToxN endoribonuclease of a).

### BRIEF DESCRIPTION OF DRAWINGS

**Figure 1****:** DNA sequence (SEQ ID NO: 3) ToxIN from E. coli encoding amino acid sequence SEQ ID NO: 1 (sequence with grey shade); Antitoxin repeat (sequence within black frame) transcribed into ToxI (SEQ ID NO: 4); terminator (sequence within dotted line).
**Figure 2****:** Profiling ToxN endoribonuclease activity: A: MW DNA ladder, B: 120 nM ToxN, C: 60 nM ToxN, D: 30 nM ToxN; E: 15 nM ToxN, F: 8 nM ToxN, G: 4 nM ToxN, H: 2 nM ToxN, I: 0 nM ToxN
**Figure 3****:** Profiling ToxN endoribonuclease activity: (1) 50 mM Tris-HCl pH 7.0, (2) 50 mM Tris-HCl pH 7.5, (3) 50 mM Tris-HCl pH 8.0, (4) 50 mM Tris-HCl pH 9.0; (A) MW DNA ladder; (B) control without ToxN; (C) 0 mM NaCl; (D) 25 mM NaCl; (E) 50 mM NaCl, (F)75 mM NaCl; (G) 100 mM NaCl; (H)125 mM NaCl; (I) 150 mM NaCl, (J) 175 mM NaCl.
**Figure 4****:** Profiling ToxN endoribonuclease activity: (A): MW DNA ladder, (B): w/o ToxN, (C): 0mM MgCl₂, (D): 1mM MgCl₂; (E): 2mM MgCl₂, (F): 3mM MgCl₂, (G): 4mM MgCl₂, (H): 5mM MgCl₂, (I): 6mM MgCl₂, (J): 7mM MgCl₂
**Figure 5****:** Profiling ToxN endoribonuclease activity: (A): MW DNA ladder, (B): w/o ToxN, (C): ToxN, (D): 5mM MgCl₂, (E): 5mM EDTA, (F): 5mM DTT 5mM, (G): 5mM MgCl₂, 5 mM EDTA, (H): 5mM MgCl₂, 10 mM EDTA, (I): 5mM MgCl₂, 5 mM EDTA, 5mM DTT (J): 5mM MgCl₂, 10 mM EDTA, 5mM DTT
**Figure 6****:** Profiling ToxN endoribonuclease activity: (A): MW DNA ladder, (B): w/o ToxN, (C): 5 min., (D): 10 min.; (E): 15 min., (F): 20 min., (G): 30 min., (H): 40 min., (I): 50 min., (J): 60 min.
**Figure 7****:** Profiling ToxN endoribonuclease activity: (A): MW DNA ladder, (B): w/o ToxN, (C): 6 °C, (D): 7 °C; (E): 10 °C, (F): 13 °C, (G): 17 °C, (H): 22 °C, (I): 27 °C.
**Figure 8****:** Profiling ToxN endoribonuclease activity: (A): MW DNA ladder, (B): w/o ToxN, (C): 25 °C, (D): 25.2 °C; (E): 26 °C, (F): 27 °C, (G): 29 °C, (H): 31 °C, (I): 33 °C, (J): 33.6 °C, (K): 34 °C, (L): 35 °C, (M): 35.7 °C, (N): 36 °C.
**Figure 9****:** illustrating the concept of determining 5'-capping efficiency of mRNA transcripts using a ToxN endoribonuclease.

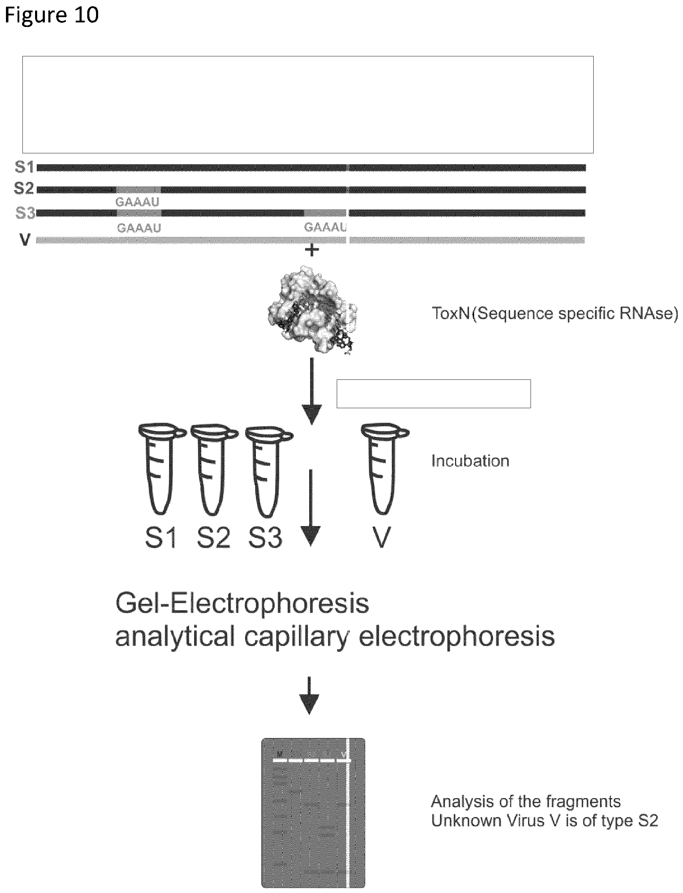

**Figure 10****:** illustrating the concept of mRNA-fingerprinting of an unknown virus strain in a sample. S1, S2, S3: RNA samples isolated from three different RNA viruses with unique distribution of recognition sites for ToxN. V: RNA sample isolated from unknown RNA virus. Analysis of fragment distribution by electrophoresis illustrates that the unknown virus (V) is a type (S2) virus.
**Figure 11**: illustrates the concept of RNA production by rolling circle transcription (RCT). (1) DNA plasmid for mRNA production, (2) single strand plasmid, (3) start of the RNA polymerase with a ToxN recognition site, (4) amplification of the RNA, once the circle is completed, (5) the RNA will continue to amplify RNA, (6) long chains of RNA with multiple copies of the mRNA of interest, (7) the RNA is cleaved by a ToxN endoribonuclease to yield multiple copies of the mRNA. This process can be simultaneous with RNA polymerase to constantly produce new mRNAs.

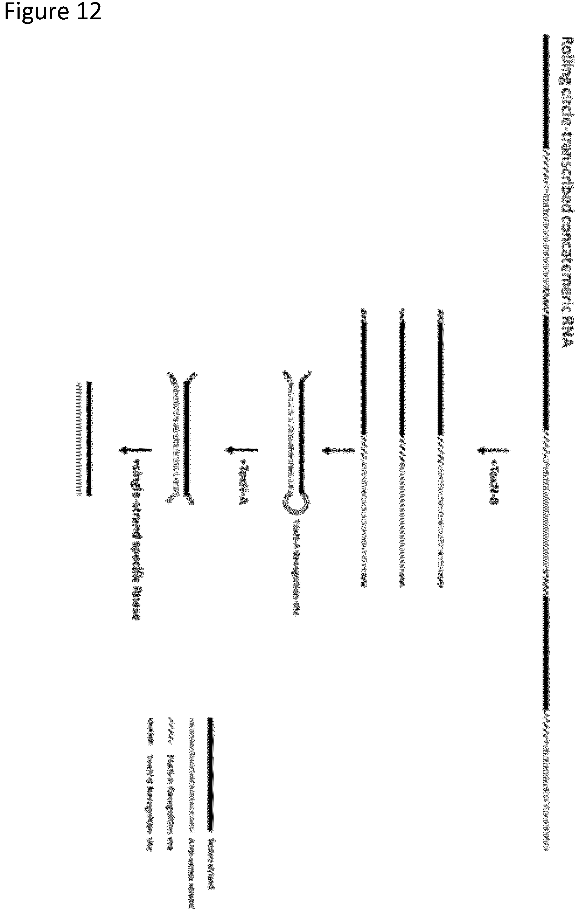

**Figure 12****:** illustrates rolling circle transcribed concatemeric RNA sequences to produce siRNAs. The transcribed sequence comprising ToxN recognition sequences for two different ToxN enzymes, ToxN-A and ToxN-B. The ToxN-B endoribonuclease will digest the RNA between pairs of concatemeric RNA sequences. Concatemeric RNA hybridize to form a hairpin structure. The ToxN-A enzyme cleaves RNA at its recognition site in the hairpin loop, the 5'-end and 3'end of the concatemeric RNA are digested with a single-strand specific RNase to remove the rest of the ToxN recognition sequences thereby producing a siRNA.

### DETAILED DESCRIPTION

Unless specifically defined herein, all technical and scientific terms used have the same meaning as commonly understood by a skilled artisan in the fields of genetics, biochemistry, molecular biology.

Where a numerical limit or range is stated herein, the endpoints are included. Also, all values and sub ranges within a numerical limit or range are specifically included as if explicitly written out.

In the following description, various examples and embodiments of the invention are set forth in order to provide the skilled person with a more thorough understanding of the invention. The specific details described in the context of the various embodiments and with reference to the attached drawings are not intended to be construed as limitations.

### Definitions:

"A polyribonucleotide" refers to a polymeric form of ribonucleotides. In some aspects, a polyribonucleotide consists of ribonucleotides only. In other aspects, a polyribonucleotide comprises ribonucleotides, and one or more modified ribonucleotides, but does not include any deoxyribonucleotides. In other cases, a polyribonucleotide comprises ribonucleotides, and may comprise one or more modified ribonucleotides, and one or more deoxyribonucleotides (including modified deoxyribonucleotides).

The terms "ribonucleic acid", RNA, "polyribonucleotide" are used interchangeably and refer to a polymeric form of ribonucleotides of any length.

"Isolated" refers to a protein or nucleic acid that, if naturally occurring, is in an environment different from that in which it may naturally occur. "Isolated" is meant to include proteins or nucleic acids that are within samples that are substantially enriched for the protein or nucleic acid of interest and/or in which the protein or nucleic acid of interest is partially or substantially purified. Where the protein or nucleic acid is not naturally occurring, "isolated" indicates the protein or nucleic acid has been separated from an environment in which it was made by either synthetic or recombinant means.

An endoribonuclease cleaves either a single- or double- stranded RNA. The endoribonucleases described in this patent application cleaves single- stranded RNA.

The terms "RNA digestion" or "RNA cleavage" are used interchangeably and refers to hydrolysis of phosphodiester bonds within a polyribonucleotide backbone in a sample.

The method of RNA digestion involves contacting a sample comprising single stranded RNA with a ToxN endoribonuclease under conditions which permit the digestion of at least a portion of the single stranded RNA present in the sample. The ToxN endoribonucleases are sequence specific and will completely digest a polyribonucleotide at their target site under sufficient time and reaction conditions that permit enzyme function. A digestion of at least a portion of the single stranded RNA present in the sample may numerically be expressed as at least 20%, 30%, 40%, 50%, 60%, 70%, 80%, 85%, 90%, 95% or 99%. Alternatively, 100% of the single stranded RNA present in the sample is digested, i.e. a complete digestion at their target site of all the RNA molecules present in the sample.

Endoribonucleases may cleave sequences which are similar to their target recognition sequence. This non-specific activity has been termed "star-activity" and should preferably be avoided. Star activity results from the recognition and cleavage of secondary cleavage sites in addition to a primary cleavage site. The secondary cleavage sites differ by one or more ribonucleotides from the primary recognition site. The star activity is characterised by the appearance of further bands in a gel electrophoresis which appear in addition to the band pattern of the complete digestion arising by specific cleavage.

The terms "contacting", "contact", "applying to", "application", "adding to" and "addition" have their ordinary meanings and are used interchangeably herein.

The present invention also covers the exact terms, features, values and ranges etc. in case these terms, features, values and ranges etc. are used in conjunction with terms such as about, around, generally, substantially, essentially, at least etc. (i.e. , "about 3" shall also cover exactly 3 or "essentially free" shall also cover "free of/ without").

The term "at least one" should be understood as meaning "one or more", and therefore includes both embodiments that include one or multiple components.

ToxN is a family of endoribonuclease from Type III toxin-antitoxin systems. The toxin (ToxN) is a protein/enzyme, while the antitoxin (ToxI) consists of multiple repeats of RNA. The toxic effects of the protein are neutralized by the specific antitoxin RNA sequence (ToxI). The toxin assembles with the individual antitoxin repeats into a cyclic complex in which the antitoxin forms a pseudoknot structure. This RNA antitoxin binds tightly to the toxin to form a hetero tetrameric or hetero hexameric cyclic, unique self-closing RNA-protein complexes, in which the toxin and antitoxin are arranged alternately in a 1:1 ratio, ToxIN complex. Identification and functional characterization of the structure of the ToxN-antitoxin (RNA) complex from E.coli is described in Manikandan, P. et al., Identification, functional characterization, assembly and structure of ToxIN type III toxin-antitoxin complex from E.coli, Nucleic acid research, 2022, vol.50, no.3, p.1687-1700.

"ToxN" (endoribonuclease)," ToxI" (antitoxin RNA molecule/inhibitor of ToxN) and "ToxIN" (heteromeric protein/RNA complex of ToxN with ToxI).

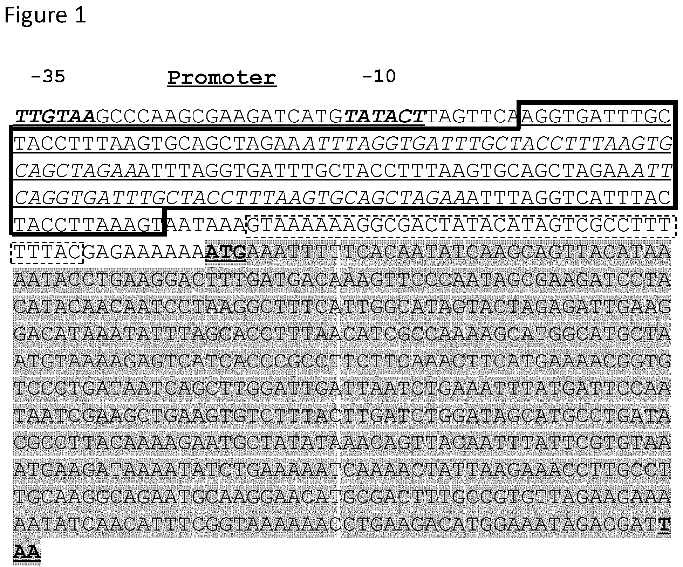

The DNA sequence encoding a ToxN endoribonuclease from E. coli, (NCBI accession number: PDB: 7D8O_A) and which is neutralized by a RNA antitoxin ToxI and form together the inactive ToxIN complex is shown in figure 1 and SEQ ID NO: 3.

The number of ribonucleotides in the recognition sequence for the ToxN endoribonucleases may vary. The recognition sequence may be from 2 to 20 ribonucleotides. Preferably the recognition sequence is from 3 to 15 ribonucleotides, more preferably 4 to 10 ribonucleotides, 4 to 9 ribonucleotides or 4 to 6 ribonucleotides.

A non-limiting example of ToxN endoribonucleases and their recognition sequence are shown in table 1 below:

**Table 1**

| **NCBI acc. No. / UniProt** | **Homolog ToxN endoribonucleases** | **SEQ ID NO** | **Recognition sequence in ssRNA** |
|---|---|---|---|
| PDB: 7D8O_A | ToxN E. coli | 1 | 5' ... GAA^AU..3' |
| B8X8Z0 | ToxN Pectobacterium atrosepticum | 6 | 5'..GAA^AU...3' |
| Q3YN09 | ToxN Bacillus thuringiensis subsp. kurstaki | 7 | 5'..AAA^AA..3' |
| Q9ZJ19.1 | ToxN (AbiQ), Lactococcus lactis subsp. lactis | 8 | 5'...AA^AA..3' |
| CBK89509.1 | [Eubacterium] rectale | 9 | 5'..GA^AAG..3' |

As mentioned above, the inventors have surprisingly and for the first time shown that a sequence specific endoribonuclease ToxN from Type III toxin-antitoxin systems cleaves single stranded RNA specifically at its recognition site in the presence of particular concentrations of a monovalent salt i.e. unspecific catalytic activity (also called star-activity") of the enzyme is reduced or absent at particular concentrations of a monovalent salt.

The inventors have also for the first time determined that, surprisingly, the unspecific catalytic, i.e. its star-activity of ToxN is reduced or absent at low concentrations or without divalent metal cations, preferably Mg²⁺ or Mn²⁺, present in the composition or sample. Free divalent cation is to be understood as not bound to a divalent ion chelator such as EDTA or EGTA. The ToxN catalytic activity is also inhibited by concentrations of divalent cations above a certain concentration. Without being bound by the theory the divalent metal cations such as Mg2+ or Mn2+ present in the composition or sample may be bound to RNA thereby inhibiting the ToxN access to its target site.

The inventors have also shown that, surprisingly, it is not necessary to remove divalent metal cations from a composition or sample comprising a ToxN endoribonuclease, rather the sequence specific catalytic activity may be maintained by a divalent cation chelator such as EDTA or EGTA.

By "essentially free of divalent metal cations" in the context of a divalent ion chelating agent is meant that the ratio of the concentration of a divalent cation, preferably Mg2+ or Mn2+ to the concentration of divalent ion chelating agent, e.g. EDTA or EGTA, in the sample is from 3:1 to 1:10, such as (e.g. Mg²⁺ or Mn²⁺ : EDTA or EGTA). Meaning that the ToxN enzyme tolerates a low concentration of a divalent metal cation present in the sample that is not bound to a divalent ion chelator without losing its catalytic activity.

This has the advantage that enzymes used in upstream applications and which requires a divalent metal cation such as Mg²⁺ or Mn²⁺ for optimal enzyme activity may be inactivated by the addition of a divalent ion chelator, e.g. EDTA or EGTA, and no subsequent purification step would be needed before the addition of ToxN, as ToxN catalytic activity and specificity is maintained when the composition or sample is essentially without free divalent cations, i.e. divalent cations not bound to an divalent ion chelator.

A further advantage is that EDTA or EGTA present in the sample may not need to be removed as the catalytic activity and specificity of the ToxN enzyme is not significantly affected by the presence of a divalent ion chelator, e.g. EDTA or The composition comprising isolated ToxN does not comprise a ToxIN complex.

The composition or sample comprising an isolated ToxN endoribonuclease or an enzymatically fragment of the ToxN may not comprise monovalent salts.

The composition or sample comprising an isolated ToxN endoribonuclease or an enzymatically fragment of the ToxN may not comprise free divalent metal cations.

The ToxN endoribonuclease may be a ToxN endoribonuclease from E. coli or an enzymatically active fragment thereof.

The expression "an enzymatically active fragment thereof" of the ToxN endoribonuclease is to be understood to mean a ToxN endoribonuclease wherein the catalytic activity of the endoribonuclease is maintained in the truncated form. Example 3 provide a suitable assay to measure endoribonuclease activity.

The ToxN endoribonuclease is preferably the ToxN with an amino acid sequence of SEQ ID NO: 1 (NCBI Acc. No.: PDB: 7D80_A) or an amino acid sequence which is at least about 70% identical to SEQ ID NO: 1.

The ToxN endoribonuclease may be a ToxN endoribonuclease or an enzymatically fragment thereof wherein the ToxN comprises an amino acid sequence which is at least 75%, 80%, 85%, 90%, 92%, 94%, 95%, 98% or 99% identical to SEQ ID No.1.

By "at least about 70%" it is meant that the sequence identity may be at least 69%, 69.5% or 69.9%. In preferred embodiments the ToxN endoribonuclease of the invention has an amino acid sequence which is at least 80%, preferably at least 85%, 90% or 95%, e.g. at least 98% or 99% or 99.5%, identical to SEQ ID No.1.

The ToxN endoribonuclease may consists of the amino acid sequence of SEQ ID No 1. Enzymatically active fragments thereof are also provided.

An endoribonuclease having an amino acid sequence which is at least 70% identical to SEQ ID No.1 may be obtained from a prokaryotic organism.

There is described a composition comprising a ToxN endoribonuclease or an enzymatically active fragment thereof, said ToxN endoribonuclease having an amino acid sequence selected from:
(a) SEQ ID No. 6 or an amino acid sequence which is at least 70% identical thereto,
(b) SEQ ID No. 7 or an amino acid sequence which is at least 70% identical thereto,
(c) SEQ ID No. 8 or an amino acid sequence which is at least 70% identical thereto, or
(d) SEQ ID No. 9 or an amino acid sequence which is at least 70% identical thereto.

In preferred embodiments, the ToxN endoribonuclease has an amino acid sequence which is at least 75%, preferably at least 80%, 85%, 90% or 95%, e.g. at least 98% or 99% or 99.5%, identical to SEQ ID Nos. 6, 7, 8 or 9. In other embodiments the ToxN endoribonuclease consists of an amino acid sequence selected from the group consisting of SEQ ID Nos. 6, 7, 8 and 9. Enzymatically active fragments thereof are also provided.

Variants of SEQ ID No. 6, 7, 8, 9 include amino acid sequences in which one or more amino acids of said amino acid sequences have undergone conservative substitutions. Preferably such substitutions are silent substitutions in that the modified form of the ToxN endoribonucleases of the invention have the same enzymatic activity as the unmodified form.

As used herein, both in respect of proteins and nucleic acid molecules or fragments thereof, when referring to "sequence identity", a sequence having at least x% identity to a second sequence means that x% represents the number of amino acids or nucleotides in the first sequence which are identical to their matched amino acids or nucleotides of the second sequence when both sequences are optimally aligned via a global alignment, relative to the total length of the second amino acid or nucleotide sequence. Both sequences are optimally aligned when x is maximum. The alignment and the determination of the percentage of identity may be carried out manually or automatically.

The skilled person will acknowledge that alignment for purposes of determining percentage sequence identity can be achieved in various ways, for instance, using publicly available computer software such as Clustal W (Thomson et al., 1994, Nucleic Acid Res., 22, pp 4673-4680) https://www.ebi.ac.uk</Tools/msa/clustalw2/ or NCBI BLAST (from National Center for Biotechnology Information (NCBI), USA) with default parameters.

Preferably the composition is a solution, preferably an aqueous solution. The term "solution" as used herein means a liquid mixture in which one or more minor components (solutes) are uniformly distributed within a major component (solvent). Typically, the minor component (solute) of a solution is soluble in the major component (solvent).

Preferably, the major component, i.e. solvent, i.e. liquid phase, is water. Preferably, the solution comprises water. The solutions of the present invention comprise at least a ToxN endoribonuclease or an enzymatically active fragment thereof as a minor component.

In a preferred embodiment, the solution is a reagent for application to a sample comprising one or more RNA molecules. Such a reagent is applied to a sample in order for the ToxN endoribonuclease in said reagent to digest said one or more ribonucleotides present in the sample. Preferably, the sample comprises multiple ribonucleotides. In this embodiment, the solution comprises a ToxN endoribonuclease or enzymatically active fragment thereof.

The term "sample" refers to a composition comprising single stranded RNA molecules.

The composition also comprises a buffer. Suitable buffers are well known in the art and any such buffer may be used. It would be within the competencies of a skilled person in the art to identify a suitable buffer.

The buffer has a buffering range of about pH 5.5 to about pH 9, preferably about pH 6.5 to about pH 9, preferably about pH 7 to about pH 9, more preferably about pH 7 to about pH 8.5.

The buffer may be Tris, HEPES or phosphate buffer. Preferably, the buffer is present in the composition or sample at a concentration of 1mM to 200 mM, preferably 10 mM to 200 mM, preferably 20 mM to 150 mM, more preferably 25 mM to 100 mM.

If present, preferably Tris-HCI is present at a concentration of 25 mM to 150 mM, more preferably 40 mM to 100 mM, more preferably about 50 mM.

The samples may have a volume of > 0.1 µl. Preferably the samples of the invention have a volume of from about 0.1 µl to about 500 µl, such as from about 0.1 µl to about 300 µl, such as from about 0.1 µl to about 250 µl, such as from about 0.1 µl to about 200 µl, such as from about 0.1 µl to about 150 µl, such as from about 0.1 µl to about 100 µl, such as from about 0.1 µl to about 75 µl, such as from about 0.1 µl to about 50 µl.

The skilled person is able to determine the appropriate concentration of the enzyme to include in the sample and the reaction mixture in order to obtain digestion of preferably all RNA molecules in the sample and at the same time avoiding unspecific digestion and star activity.

The reaction mixture may comprise further components that may be present due to having been added at an earlier stage of the workflow and tolerable in the reaction assay, e.g. DTT, nucleotides, S-adenosylmethionine (SAM) or other enzymes. DTT is a reducing agent that stabilizes disulphide bonds within enzymes and thereby stabilizing the enzyme structure.

As described above the composition or sample comprising ToxN endoribonuclease or an enzymatically active fragment thereof comprises preferably a specific concentration of a monovalent salt.

The monovalent salt present in the composition or sample may selected from inorganic salts comprising alkali metal ions wherein the alkali metal ions are selected from Na+, K+, Li+, Rb+, Cs+ and Fr+ or any combinations thereof.

Thus, preferably the monovalent salt is an alkali metal salt.

Preferably the alkali metal ions of the salt are selected from Na+, K+, Li+ and Rb+.

The anions of the salts comprising alkali metals ions are preferably selected from fluorine (F), chlorine (Cl), bromine (Br), iodine (I), sulphates, phosphates or hydroxides or any suitable combinations thereof.

Preferably the alkali metal salts are NaCl, KCl, Na2SO4, K2SO4, KOH, NaOH, Na-Phosphates, K-Phopshates or any suitable combinations.

Thus, preferably the composition or samples comprising a particular concentration of a monovalent salt is referred herein to a composition or sample that comprises a concentration of monovalent salt ≤ 150 mM.

The term "about ≤ X mM" is equivalent to from 0 to about X mM".

The composition or sample may not comprise a monovalent salt.

Thus, in one aspect the composition or sample comprising a ToxN endoribonuclease or an enzymatic fragment thereof comprises a concentration of alkali metal salt in the composition or sample <150 mM, about ≤100 mM, about ≤75 mM, about ≤55 mM, from about 20 mM to about 75 mM, from about 20 mM to about 55 mM.

As described above the compositions or samples comprising a ToxN endoribonuclease or an enzymatic fragment thereof are essentially without free divalent metal cations, i.e. the composition or sample may comprise low concentration of a divalent metal cation. Free divalent metal cations are referred herein to divalent metal cations not bound to a divalent cation chelator such as EDTA or EGTA.

Thus, the compositions or samples may comprise a concentration of free divalent metal cation about ≤3 mM.

The composition or samples may be without free divalent metal cations.

The free divalent cations are preferably selected from Mg²⁺ and Mn²⁺.

The composition or samples may be without free Mg²⁺.

The composition or samples may be without free Mn²⁺.

Preferably, the concentration of free Mg²⁺ and/or free Mn²⁺ in the compositions and samples are about ≤ 2 mM, more preferably about ≤ 1 mM.

The composition or sample comprising a ToxN endoribonuclease or an enzymatic fragment thereof may comprise a concentration of free Mg²⁺ and/or Mn²⁺ in the composition or sample in the range from 0 to about 1 mM, from about 1 µM to about 1 mM, from about 1 µM to about 0.9 mM, from about 1 µM to about 0.8 mM, from about 1 µM to about 0.7 mM, from about 1 µM to about 0.6 mM, from about 1 µM to about 0.5 mM.

The composition or sample comprising a ToxN endoribonuclease or an enzymatic fragment thereof may comprise a ratio of concentration of a divalent metal cation to a concentration of a divalent ion chelating agent in the composition or sample such that the max concentration of free divalent cation in the composition or sample, i.e . not bound to a divalent ion chelator is not above 3 mM, preferably not above 2 mM and more preferably not above 1 mM

The composition or sample comprising a ToxN endoribonuclease or an enzymatic fragment thereof may comprise a concentration of a divalent ion chelator of about ≤ 10 mM.

The divalent ion chelator is preferably EDTA or EGTA.

For solubility reasons the compositions and samples comprising divalent metal cations are preferably added as a divalent salt. A divalent salt is a salt in which at least one of the counter ions is divalent, e.g. MgCl₂ or MnCl₂. The salts are preferably inorganic.

An inorganic salt is a salt in which neither of the counter ions comprises carbon. The mono- or divalent salts are preferably inorganic salts.

Preferably the compositions and samples comprise monovalent salts and preferably also monovalent anions which are counterions. The preferred concentrations of monovalent salts disclosed herein are, inherently, the preferred concentrations of monovalent counterions, and vice versa.

Thus, there is provided herein a composition or a sample comprising a ToxN endoribonuclease or an enzymatically active fragment thereof, said ToxN endoribonuclease comprises amino acid sequence of SEQ ID No.1 or comprising an amino acid sequence which is at least 70% identical to SEQ ID No.1 wherein
- concentration of monovalent salt in the composition or sample is about ≤100 mM, about ≤75 mM, about ≤55 mM, from about 20 mM to about 75 mM, preferably from about 20 mM to about 55 mM.

It is also provided a composition or a sample comprising a ToxN endoribonuclease or an enzymatically active fragment thereof, said ToxN endoribonuclease comprises amino acid sequence of SEQ ID No.1 or comprising an amino acid sequence which is at least 70% identical to SEQ ID No.1 wherein
- concentration of monovalent salt in the composition or sample is about ≤100 mM, about ≤75 mM, about ≤55 mM, preferably from about 20 mM to about 75 mM, preferably from about 20 mM to about 55 mM
   and wherein
- the composition or sample is essentially without free divalent metal cations wherein the divalent metal cations are preferably Mg2+ or Mn2+ and wherein the divalent metal cations are provided as inorganic salts, such as MgCl2 or MnCl2.

There is also provided a composition or a sample wherein the ToxN endoribonuclease or an enzymatically active fragment thereof, said ToxN endoribonuclease comprises amino acid sequence of SEQ ID No.1 or comprising an amino acid sequence which is at least 70% identical to SEQ ID No.1 wherein
- concentration of monovalent salt in the composition or sample is about ≤100 mM, about ≤75 mM, about ≤55 mM, preferably from about 20 mM to about 75 mM, preferably from about 20 mM to about 55 mM
   and wherein
- concentration of free divalent metal cation is about ≤1 mM, the divalent metal cations are preferably Mg²⁺ or Mn²⁺ and wherein the divalent metal cations are provided as inorganic salts such as MgCl₂ or MnCl₂.

There is also provided a composition or a sample wherein the ToxN endoribonuclease or an enzymatically active fragment thereof, said ToxN endoribonuclease comprises amino acid sequence of SEQ ID No.1 or comprising an amino acid sequence which is at least 70% identical to SEQ ID No.1 wherein
- concentration of monovalent salt in the composition or sample is about ≤100 mM, about ≤75 mM, about ≤55 mM, preferably from about 20 mM to about 75 mM, preferably from about 20 mM to about 55 mM.
   and wherein
- the composition or sample comprises ratio of concentration of a divalent metal cation to the concentration of divalent ion chelating agent in the composition or sample such that the concentration of free divalent metal cation present in the sample or composition is about ≤1 mM and the concentration of divalent ion chelator is about ≤10 mM
- the divalent metal cations are preferably Mg²⁺ or Mn²⁺ and provided as inorganic salts such as MgCl₂ or MnCl₂ and
- the divalent ion chelating agent is preferably EDTA or EGTA.

As explained above free Mg²⁺ or Mn²⁺ in the sample is denoted herein as not bound to EDTA or EGTA.

Thus for example the ratio of concentration of a divalent metal cation to the concentration of divalent ion chelating agent in the composition or sample may be for example 2 mM : 1 mM, 5mM : 5mM, 5mM : 10mM, 10 mM : 10 mM or any other alternative ratio combinations providing that the concentration of free divalent metal cation in the composition or sample is about ≤1 mM and the concentration of divalent ion chelator is preferably about ≤10 mM.

### Preparation of the ToxN endoribonuclease

A method of preparing ToxN endoribonuclease is described in Manikandan, P. et al., Identification, functional characterization, assembly and structure of ToxIN type III toxin-antitoxin complex from E.coli, Nucleic acid research, 2022, vol.50, no.3, p.1687-1700 and further described in Example 1.

The ToxN endoribonuclease and the enzymatically fragments thereof or nucleic acid molecules encoding the endoribonuclease may be isolated from a natural source such as a bacteria, for example E. coli, Pectobacterium atrosepticum, Bacillus thuringiensis subsp. Kurstaki, Lactococcus lactis subsp. lactis or [Eubacterium] rectale.

Alternatively, the enzyme may be produced recombinantly in a host cell and isolated and purified therefrom. Wherein the host cell is not, or not from an organism which naturally express the gene encoding the ToxN endoribonuclease, i.e. the host cell is a heterologous host cell such as a yeast cell, an insect cell, a human cell line or a bacterial cell, preferably E. coli.

Nucleic acid sequences encoding the ToxN endoribonuclease or enzymatically fragments thereof according to the present invention may be amplified using PCR from genomic DNA, isolated as a cDNA or may be ordered by a commercial supplier such as GENEWIZ, GeneArt from Thermo Fisher Scientific or Genscript.

As described above the nucleic acid sequence encoding the ToxN endoribonuclease or enzymatically fragments thereof may be codon optimized for increased protein production in a heterologous host cell. A variety of software programs for help with codon-optimization are well known in the art. CodonW is an example of an open source software program that may be used. Preferably the GeneOptimizer algorithm described by Raab, D., Graf, M., Notka, F., Schödl, T., & Wagner, R. (2010). The GeneOptimizer Algorithm: Using a sliding window approach to cope with the vast sequence space in multiparameter DNA sequence optimization. Systems and Synthetic Biology, 4(3), 215-225, is used for generating a codon optimized DNA sequence for expression of the ToxN endoribonuclease in a E. coli host cell.

There are various available molecular techniques for expression of proteins from DNA sequences by heterologous expression in various host cell systems using well known recombinant gene expression systems. For example, the nucleic acid molecule encoding a ToxN endoribonuclease or encoding an enzymatically fragment thereof may be inserted in a suitable expression vector comprising necessary transcriptional and translational elements for expression that are appropriate for the chosen host cell. Example of commonly used expression vectors are plasmids or viruses.

To ensure a reliable transcription of the gene of interest. The expression vector may comprise a strong promoter, bacteriophage T5 and T7 are examples of strong promoters for expression in E. coli. The promoter may be regulated by comprising chemical switches. Example of inducible promoters for use in E. coli is the commonly used lac promoter induced by Isoropyl-beta-D-thiogalactoside (IPTG) (Hansen LH, Knudsen S, Sørensen SJ, "The effect of the lacY gene on the induction of IPTG inducible promoters, studied in Escherichia coli and Pseudomonas fluorescens", Curr. Microbiol. 1998, 36 (6): 341-7) or the XylS/Pm expression cassette comprising a promoter that is inducible with toluic acid (Gawin, A. et al., The XylS/Pm regulator/promoter system and its use in fundamental studies of bacterial gene expression, recombinant protein production and metabolic engineering, Microb. Biotechnol., 2017, Vol. 10, No.4, p 702-718). The XylS/Pm regulator/promoter system originating from the Pseudomonas putida is widely used for regulated low- and high-level recombinant expression of genes and gene clusters in E. coli and other bacteria.

A further aspect of the invention is a method of expression of a ToxN endoribonuclease or an enzymatically fragment thereof as described above in a suitable heterologous cell. The host cell may be a bacterium or a yeast cell. Preferably the expression of the enzyme is in a bacterial host cell, more preferably E.coli, BL21 (DE3) cells.

Transformation of the above described expression vector comprising the ToxN endoribonuclease may be performed by methods well known to a skilled person, e.g. by using chemically competent cells.

As described above, the ToxN endoribonuclease may be synthesized using recombinant DNA technology. Alternatively, the endoribonuclease may be produced using a cell-free expression system or chemical synthesis of a ToxN endoribonuclease.

A ToxN endoribonuclease enzyme comprising a signaling peptide for secretion into cell culture media may be isolated and purified from the host cell culture media using any technique known in the art and well described in literature. Examples of such techniques or any combination may include precipitation, ultrafiltration, different chromatographic techniques e.g. size-exclusion chromatography, immobilized metal affinity column chromatography and/or immunoadsorption chromatography.

A ToxN endoribonuclease produced intracellularly may be isolated and purified also using techniques well known to a skilled person. Examples of methods for preparation of cell lysates from E. coli cells are homogenization, sonication or enzymatic lysis using lysozyme. After the ToxN endoribonuclease is released from the lysed cells the enzyme may be subject to any method of purification for example size-exclusion chromatography, immobilized metal affinity column chromatography and/or immunoadsorption chromatography.

The ToxN endoribonuclease may comprise a C-terminal or N-terminal His-tag to ease isolation, purification and/or identification of the enzyme. An N-terminal poly-histidine tagged ToxN endoribonuclease is depicted in SEQ ID No. 2.

The purified ToxN endoribonuclease or an enzymatically fragment thereof may finally be stored in a buffer.

The purified ToxN endoribonuclease enzyme or an enzymatically fragment thereof may finally be stored in a suitable buffer. Suitable buffers for storing the ToxN endoribonuclease is known to a skilled person.

### Kits comprising ToxN endoribonucleases

The compositions and samples of described herein comprises an endoribonuclease that is not inhibited by the presence of a divalent ion chelator, which is often added to enzyme reactions to stop the catalytic activity of an enzyme. The ToxN endoribonucleases therefore have advantageous utility in various molecular biology methods, which involve prior use of other enzymes. Such methods are discussed in more detail below. Thus, in a further aspect there is provided a kit comprising:
i) a composition comprising a ToxN endoribonuclease as defined above; and
ii) a second composition comprising a second enzyme selecting from a group consisting of a RNA polymerase, a RNA ligase for ligating single stranded RNA molecules, a pyrophosphatase, a phosphatase, a kinase or any other nucleic acid or ribonucleic acid modifying enzymes and at least one further ToxN endoribonuclease with a different recognition site from the ToxN endoribonuclease of a).

The second enzyme may be another ToxN endoribonuclease having a different recognition site compared to the ToxN enzyme as mentioned in i).

Kits comprising a ToxN endoribonuclease may comprise a suitable buffer for carry out digestion of RNA.

Suitable RNA digestion buffers and reaction conditions for carry out cleavage of RNA is described in detail above.

### Method of cleaving single stranded RNA

The enzymatic activity of the ToxN endoribonucleases makes such enzymes especially suited for use in methods that involves cleavage of single stranded RNA.

The different ToxN endoribonucleases cleave single stranded RNA molecules at different recognition sites.

Thus, in one aspect the composition comprising a ToxN endoribonuclease may be a solution for application to a sample wherein the sample comprising at least one isolated single stranded RNA molecule comprising at least one cleavage site for a ToxN endoribonuclease.

There is also described a method of cleaving single stranded RNA molecules in a sample, wherein the method comprises the steps:
a. providing a sample comprising at least one single stranded RNA molecule comprising at least one cleavage site for a ToxN endoribonuclease; and
b. contacting a ToxN endoribonuclease or an enzymatically active fragment thereof with said sample under conditions which permits cleavage of at least a portion of said RNA molecules present in the sample.

Preferably the ToxN endoribonucleases are the ToxN endoribonucleases as defined above.

Suitable RNA digestion buffers and reaction conditions for carry out cleavage of RNA is described in detail above.

The step of digestion will typically be incubation and is described above and in the examples. Suitable incubation comprises incubation at around 10°C to around 50°C, such as around 10°C to 30°C, preferably around 15°C for 1 minute to about 2 hours, such as from about 5 minutes to about 1.5 hours, such as from about 15 minutes to about 1 hour.

The single stranded RNA molecule in the sample may be a concatemer comprising multiple copies of precursors of either mRNA, siRNA, circular RNA, precursors, microRNA or ribozyme and wherein the concatemeric RNA molecule comprises a cleavage site for a ToxN endoribonuclease between each copy of precursors of mRNA, siRNA, circular RNA, microRNA or ribozyme.

### Method for preparing circular RNA molecules

The nature of the RNA 5'end and 3'end produced by ToxN can be exploited through subjecting the RNA to a RtcB Ligase reaction which ligates 3'-PO₄ ends with 5'-OH ends of RNA. By this reaction the RNA becomes circular. The mechanism of making circular RNA by the use of RtcB ligases is described in Tanaka, N. et al., Novel mechanism of RNA repair by RtcB via sequential 2', 3'-cyclic phosphodiesterase and 3'-phosphate/5'-hydroxyl ligation reactions, J Biol Chem., 2011, vol.286, no.50, p.43134-43143.

In a further aspect there is provided a method for preparing single stranded circular RNA molecules present in a sample wherein the RNA molecules comprise a cleavage site for a ToxN endoribonuclease; contacting a ToxN endoribonuclease with said RNA molecule under conditions that permit digestion of at least a portion of the RNA molecules present in the sample thereby producing RNA molecules comprising 3'-PO₄ ends and 5'-OH ends; contacting the digested RNA molecules with RtcB ligase under conditions which permits ligation thereby producing circular RNA.

### Method of preparing multiple copies of RNAs by rolling circle transcription (RCT)

Rolling circle transcription (RCT), using small circular single-stranded DNA as the template, has been well investigated in the past two decades. Interestingly, transcription via the rolling circle mechanism is achieved by using the T7 RNA polymerase but may happen in absence of the specific canonical promoters and generate transcripts that are tandemly repeated sequences complementary to the circular template. In the literature the use of RNase H (Wang et al., Preparation of small RNAs using rolling circle transcription and site-specific RNA disconnection, Molecular Therapy -Nucleic Acids, 2015, e215) or ribozymes (WO2020023741) are reported.

By adapting the method to the use of site specific ToxN endoribonucleases the method is simplified and no longer requires RNase H and a helper DNA fragment or the use of synthetic or in-the-transcript encoded ribozymes.

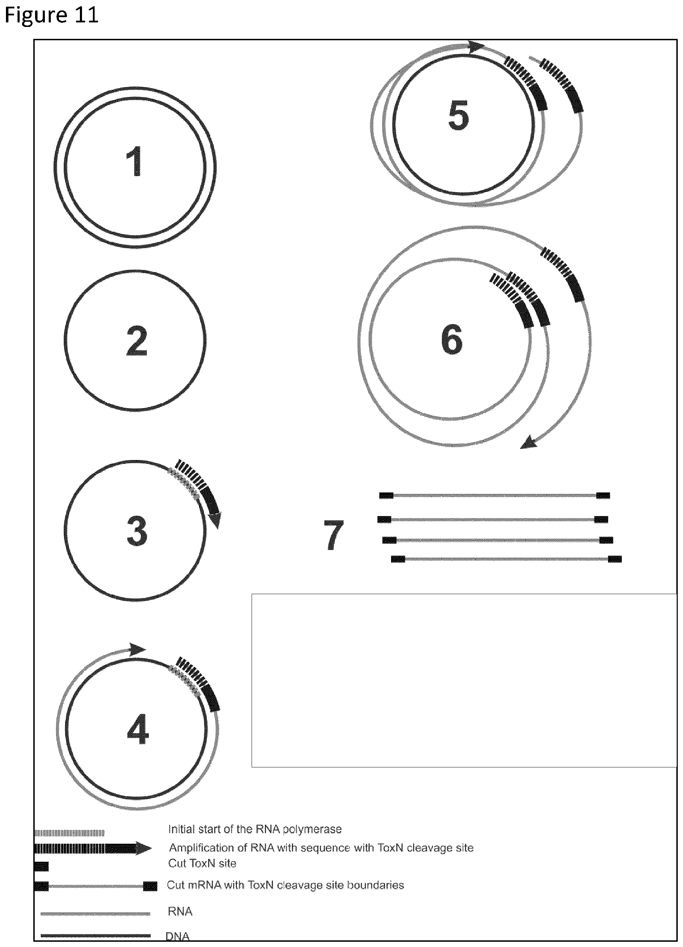

There is also described a method for synthesizing RNA by rolling circle transcription (RCT), as outlined in figure 11. The method comprises the steps:
- providing a single stranded DNA plasmid comprising a sequence encoding an RNA recognition site for a ToxN endoribonuclease optionally in proximity of a recognition site (also called promoter) for an RNA polymerase;
- amplification of the RNA;
- once the circle is completed, the RNA polymerase will continue to amplify RNA into long chains with multiple copies of the RNA of interest;
- the RNA is cleaved by ToxN to yield multiple copies of the RNA. This process can be simultaneous with RNA polymerase to constantly produce new RNAs.

Preferably the ToxN endoribonucleases are the ToxN endoribonucleases as defined above.

Suitable RNA digestion buffers and reaction conditions for carry out cleavage of RNA is described in detail above.

### Method of preparing double stranded siRNAs

Small interfering RNA (siRNA), sometimes known as short interfering RNA or silencing RNA, is a class of double-stranded RNA typically 20-24, normally around 21 base pairs in length, i.e. similar to miRNA. siRNAs operate within the RNA interference (RNAi) pathway and it interferes with the expression of specific genes with complementary nucleotide sequences by degrading mRNA after transcription, thereby preventing translation.

In a further aspect there is described a method of synthesizing siRNAs. The method comprising the steps:
a. providing a sample comprising at least one rolling circle transcribed concatemeric RNA molecule comprising cleavage sites for two different ToxN endoribonucleases, ToxN-A and ToxN-B, having different recognition sites, wherein the recognition sites for ToxN-B is situated between tandem repeats and recognition sequences for ToxN-A is situated within the tandem repeats;
b. contacting said sample with the ToxN-B endoribonuclease or an enzymatically active fragment thereof under conditions which permits cleavage of at least a portion of said RNA molecules present in the sample thereby producing single repeats that form a hairpin structure based on their sense-antisens sequence information; contacting the formed hairpin structures with a second ToxN-A enzyme that cleaves the RNA in the loop structure to form double stranded RNA molecules; and
c. the resulting overhangs containing parts of the ToxN recognition sites is removed by using a standard single-strand specific ribonuclease such as RNase T1 thereby producing double stranded siRNAs. The method is illustrated in figure 12.

Preferably the ToxN endoribonucleases are the ToxN endoribonucleases as defined above.

Suitable RNA digestion buffers and reaction conditions for carry out cleavage of RNA is described in detail above.

### Method for analyzing modifications of RNA

The family of ToxN endoribonucleases are highly useful in RNA analysis in particular of long modified RNA molecules which have to be cleaved into fragments in order to be able to aid the analysis of the RNA modification.

Similar to the well-known DNA endoribonucleases enzymes commonly used in in vitro molecular cloning methods of double stranded DNA, the family of ToxN endoribonucleases is a group of enzymes that cleave single stranded RNA at specific recognition sequences without the need of any guide RNA, ribozymes or in complex with DNA probes.

5'capping of synthetically produced mRNA is crucial for an efficient in vivo translation of the mRNA into a functional protein or peptide.

However, capping of RNA in mRNA manufacturing is known to be an incomplete process leaving a portion of the produced mRNA uncapped. Completeness of capping can be assessed by methods such as LC-MS, IP RP HPLC, gel electrophoresis such as PAGE or capillary gel-electrophoresis (CGE).

The addition of a cap-structure changes the molecular weight of an RNA fragment at a level that is undetectable in standard molecular biology analytics. In addition, heterogeneity of an RNA product solution in terms of different product lengths makes it even more difficult to assess a change in molecular weight of the whole RNA population.

Thus, contemplated length harmonization of the whole RNA population would eventually yield only 2 to 5 sub-populations differing in terms of molecular weight dependent on their capping state.

Different types mRNA capping structures are well known to a skilled person and Chan, S.H. et al., RNase H-based analysis of synthetic mRNA 5'cap incorporation, RNA 2022, vol.28, p.1144-1155 discloses several examples of 5'mRNA capping structures. Chan, S.H. et al. 2022 also discloses analysis of 5'capping efficiency of mRNA using DNA-RNA chimera-guided RNaseH cleavage of newly synthetically produced mRNA. However, a problem with RNaseH in this method is that it is difficult to get uniform cuts as the reaction has to be optimized both with regard to optimal DNA-RNA hybridization and the enzymes ability to provide a uniform cut. Another advantage with ToxN endoribonucleases is that they only require a pentamer as recognition site.

The use of ribozymes instead of RNase H has been suggested, EP3183340, however ribozymes requires excess amount of the enzyme and the enzyme is highly dependent on a 3D-structure of the RNA for cleavage.

Recently also capping of other RNA molecules has been detected in eukaryotes, bacteria and archaea. These noncanonical caps are primarily derived from metabolites and cofactors such as NAD⁺, FAD⁺ among others. The nonconical caps can affect RNA stability, mitochondrial function and RNA translation, Doamekpor et al. 2022 (https://www.ncbi.nlm.nih.gov/pmc/articles/PMC9283932/)

The inventors have shown for the first time that the family of ToxN endoribonucleases can overcome the problem of the endoribonucleases of the prior art, as the enzyme is able to digest single stranded RNA without the need of a hybridized DNA probe, a guide RNA or particular 3D structure of the RNA molecule for RNA cleavage.

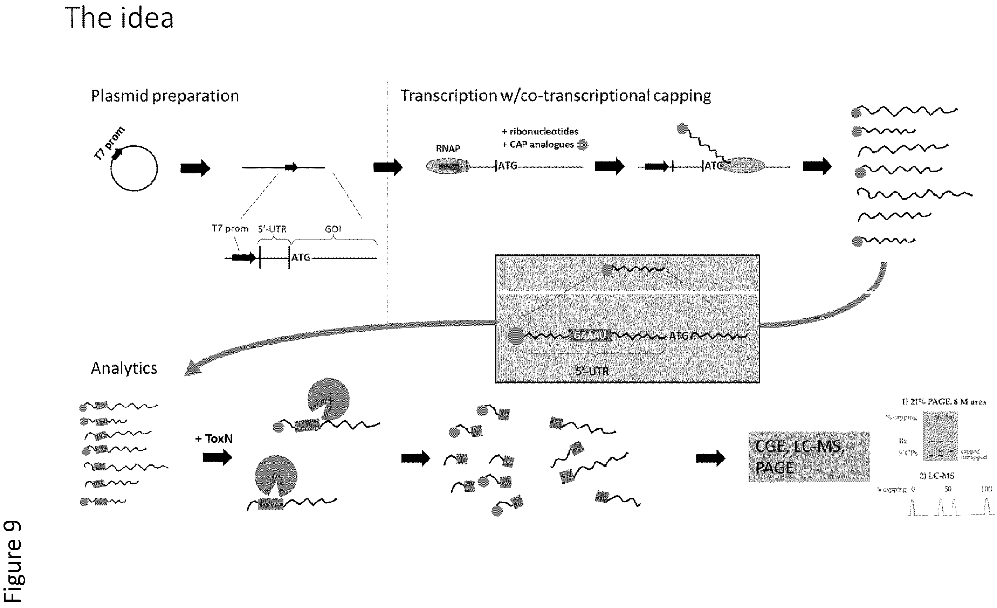

A method of analyzing the 5'capping efficiency of in vitro transcribed including co-transcriptional 5'capping of mRNA using ToxN enzyme is outlined in figure 9. The mRNA is in vitro transcribed and includes a recognition site for a ToxN enzyme in the 5'UTR.

The ToxN recognition sequence is placed at a predefined number of ribonucleotides from the 5'end of the synthetically transcribed and 5'capped mRNA. Digestion of the 5'capped RNA with ToxN produced a population of 5'capped mRNA molecules of an equal ribonucleotide length.

For analysis of modifications of mRNA, the molecular weight difference provided by the modification is insignificant in long RNA molecules.

Long RNA molecules are RNA molecules of at least 10, 100, 200, 500 or 1000 nucleotides in length. Preferably the RNA molecule has a length from 5 to 50000 nucleotides, 10 to 30000 nucleotides, 100 to 25000 nucleotides or 200 to 20000 nucleotides, 500 to 15000 nucleotides.

In one aspect the 5'capped mRNA fragment generated after digestion with a ToxN enzyme comprises 2 to 100 ribonucleotides, preferably from 5 to 50 ribonucleotides and more preferably from 5 to 10 ribonucleotides.

In an alternative method a recognition sequence for a ToxN enzyme may be transcriptionally introduced on each side of a possible RNA fragment in question in order to investigate RNA modifications at internal positions of an RNA molecule.

In an alternative method a recognition sequence for a ToxN enzyme may be transcriptionally introduced in front of the Poly(A)-tail of a possible mRNA fragment in question in order to investigate the Poly(A)-tail length distribution of an mRNA molecule.

Thus, in one aspect there is described a method of determining 5'capping efficiency of RNA molecule modification wherein the method comprises the steps:
a. providing a sample comprising at least one single stranded mRNA molecule comprising a cleavage site for a ToxN endoribonuclease in the 5'UTR of said mRNA;
b. contacting said sample from step a) with a ToxN endoribonuclease or an enzymatically active fragment thereof under conditions that permits cleavage of at least a portion of said single stranded RNA molecules to produce at least one 5' terminal RNA fragment and at least one 3' terminal RNA fragment.
c. separating the RNA fragments from step b) and determine the presence of a 5'-capping modification at the 5'end of said 5' terminal RNA fragment.

The separation and detection of step c) of the above method is based on different molecular weight, charge or length of the generated RNA fragments.

Preferably the ToxN endoribonucleases are the ToxN endoribonucleases as defined above.

Suitable RNA digestion buffers and reaction conditions for carry out cleavage of RNA is described in detail above.

There is also described a method of determining Poly(A)-tail length distribution of RNA molecules wherein the method comprises the steps:
a. providing a sample comprising at least one single stranded mRNA molecule comprising a cleavage site for a ToxN endoribonuclease in the 3'UTR of said mRNA located upstream of the Poly(A)-tail;
b. contacting said sample from step a) with a ToxN endoribonuclease or an enzymatically active fragment thereof under conditions that permits cleavage of at least a portion of said single stranded RNA molecules to produce at least one 5' terminal RNA fragment and at least one 3' terminal RNA fragment.
c. separating the RNA fragments from step b) and determine the presence and length of a Poly(A) tail at the 3'end of said 3' terminal RNA fragment.

The 3' terminal RNA fragments comprising poly(A)-tails may be enriched by using an oligo-dT based enrichment step thereby removing the non-Poly(A) containing fraction.

Preferably the ToxN endoribonucleases are the ToxN endoribonucleases as defined above.

Suitable RNA digestion buffers and reaction conditions for carry out cleavage of RNA is described in detail above.

In a further aspect the separation and detection of step c) of the above method is based on different molecular weight, charge or length of the generated RNA fragments.

In a further aspect separation and detection of the RNA fragments is selected from gel electrophoresis, capillary electrophoresis, high pressure liquid chromatography (HPLC), mass spectrometry (MS) or LC-MS, LC-UV.

### Method for RNA fingerprinting

RNA fingerprinting is an important tool within diagnostics and therapeutics. ToxN endoribonucleases for use in analytically RNA fingerprinting has several advantages including a fast and reliable fragmentation of single stranded RNA molecules of defined length that can be analysed by gel electrophoresis, capillary electrophoresis, high pressure liquid chromatography (HPLC), mass spectrometry (MS) or LC-MS, LC-UV.

There is provided a method of RNA fingerprinting, wherein the method comprises the steps:
a. providing a sample comprising a single stranded RNA molecule with unknown sequence;
b. contacting said sample from step a) with a ToxN endoribonuclease or an enzymatically active fragment thereof under conditions that permit cleavage of at least a portion of the RNA molecules present in said sample obtaining a plurality of RNA fragments;
c. separation and detection of the fragmented RNA molecules from step b) thereby obtaining a fingerprint of said RNA molecule with unknown sequence and compare the obtained fingerprint with fingerprints from RNA molecules with known RNA sequence.

Preferably the ToxN endoribonucleases are the ToxN endoribonucleases as defined above.

Suitable RNA digestion buffers and reaction conditions for carry out cleavage of RNA is described in detail above.

Determination of RNA species within a multivalent RNA composition as described in WO2022212711, is another RNA fingerprinting method wherein the use of a ToxN endoribonuclease will simplify and improve the method over RNase H enzymes that require a hybridized DNA probes at their recognition site.

The method is based on RNA compositions comprising one or more distinct RNA species (e.g, RNAs encoding different proteins), where each RNA species comprises a unique nucleotide sequence that can be used to identify the RNA species. The incorporation of unique identification and/or ratio determination (IDR) sequences into distinct RNA species of a RNA composition in at least one position inferring a unique fingerprint to each RNA species (e.g., within a non-coding region).

From WO2022212711 and without wishing to be bound by theory, it is believed that RNAs can be digested to release RNA fragments comprising the IDR sequence, and analytical methods can be used to quantify the types and amounts of RNA fragments containing each IDR, to generate a profile of the types and/or amounts of each RNA species in a RNA composition.

Use of IDR sequences for analysis allows characterization of multivalent RNA compositions comprising several distinct RNA species, even if multiple RNA species are difficult to distinguish by length or coding sequence. For example, a multivalent RNA composition comprising eight RNA species, each encoding a different serotype of the same protein, may have similar lengths and coding sequences, but each RNA species may comprise a different IDR pattern in a coding or non-coding region.

Because each IDR sequence unambiguously identifies a particular RNA species, the abundance of IDR sequences may be measured to determine the abundance of RNAs encoding each serotype.

Furthermore, the coding sequence of one or more RNA species in a multivalent RNA composition may be modified (e.g., to alter the structure of an encoded therapeutic protein or antigen) independently from the IDR sequence, such that the same analytical methods may be used to evaluate a RNA composition in which one or more RNA coding sequences are modified.

There is also described a method for analysing a RNA species in a multivalent RNA composition, wherein the method comprises the steps:
a. providing a sample comprising a multivalent RNA composition comprising a first RNA species and a second RNA species comprising a cleavage site for a ToxN endoribonuclease in at least one position of said first RNA species and a second RNA species;
b. contacting said sample from step a) with a ToxN endoribonuclease under conditions that permit cleavage of said first RNA species and a second RNA species thereby releasing a plurality of first RNA fragments and second RNA fragments from the first and second RNA species;
c. separation and detection the presence and/or amount of the released first RNA fragments and second RNA fragments.

The RNA molecule may be selected from mRNA, viral RNA, invitro transcribed mRNA, therapeutic RNA.

Preferably the ToxN endoribonucleases are the ToxN endoribonucleases as defined above.

Suitable RNA digestion buffers and reaction conditions for carry out cleavage of RNA is described in detail above.

The separation and detection of step c) of the above method may be based on different molecular weight, charge or length of the generated RNA fragments.

In a further aspect separation and detection of the RNA fragments is selected from gel electrophoresis, capillary electrophoresis, high pressure liquid chromatography (HPLC), mass spectrometry (MS) or LC-MS, LC-UV.

Having generally described this invention, a further understanding can be obtained by reference to certain specific examples. The examples illustrate the properties and effects of the of the ToxN endoribonucleases, and are provided herein for purposes of illustration only, and are not intended to be limiting.

### EXAMPLES

### Example 1 - Cloning, expression and purification of ToxN endoribonuclease with SEQ ID NO: 1

The ToxN enzyme (toxin) was cloned into pBAD/His A vector and the RNA (antitoxin) was cloned into pRSFDuet^{™}-1 as described in Manikandan, P. et al., Identification, functional characterization, assembly and structure of ToxIN type III toxin-antitoxin complex from E.coli, Nucleic acid research, 2022, vol.50, no.3, p.1687-1700. The plasmids containing toxin and antitoxin were co-transformed into E. coli BL21(DE3) cells and grown overnight at 37°C, 180 rpm, followed by the secondary culture at 37°C, 180 rpm till OD600 ~0.5. The culture was incubated at 15°C without shaking for 30 min and the toxin was induced by adding IPTG to a final concentration of 1 mM and incubated at 15°C, 180 rpm for 24 h. The cells were harvested by centrifugation at 6000 rpm for 15 min. The cells were resuspended in lysis buffer (50 mM Tris, 300 mM NaCl, 10 mM imidazole, 10% glycerol, 2 mM 2-mercaptoethanol pH 7.5 at 25°C) and lysed by sonication. The lysate was centrifuged at 13 000 rpm for 30 min, and the supernatant was loaded on a Ni²⁺ -NTA column. The complex was eluted using elution buffer (lysis buffer + 200 mM imidazole). Fractions containing the complex were dialyzed against ionexchange buffer (50 mM NaCl, 50 mM Tris-HCl, 1 mM DTT pH 7.5) and purified using anion exchange chromatography by increasing gradient of NaCl from 50 to 1000 mM, over a volume of 100 ml, which yielded separate fractions of toxin, ToxN (at ~300 mM NaCl), antitoxin, RNA (at ~600 mM NaCl) and complex of ToxN-RNA (at ~500 mM NaCl). They were further purified by size exclusion chromatography (SEC) using an S200 column (GE).
Toxin (ToxN)_Fwd 5'-AGGTCATATGGCGAAATTTTTCACAATATCA-3'
Toxin (ToxN) _Rev 5'-GGAAATAGRCGATCTCGAGTCTAGAGCAT-3'

### Example 2 - in vitro transcription and production of RNA oligoes comprising a ToxN recognition and cleavage site

In vitro transcription was done in 50 µL reactions with 30U T7 RNA Polymerase (ThermoScientific, EP0111) using the included 5X reaction buffer (200 mM Tris-HCl pH 7.9; 30 mM MgCl2, 50 mM DTT, 50 mM NaCl, 10 mM spermidine), 50U RiboLock RNase Inhibitor (ThermoScientific, EO0381), 2 mM NTPs (ThermoScientific, R1481), and 1 µg linearized (ScaI digested) pGEMEX-1 template. As alternative templates any linearized plasmids with a T7 promoter and ToxN cleavage sites or PCR products including a T7 promoter and ToxN cleavage sites can be used. The transcription reaction lasted for 2h at 37°C and was inactivated by adding 10 µL 60 mM ETDA followed by a 10 min incubation at 65°C. Reaction cleanup was conducted using the RNeasy MiniElute Cleanup Kit (Qiagen, 74204) to elute the purified RNA in RNase free water.
Recognition sites: E.coli ToxN: GAA^AU
P. atrosepticum ToxN: GAA^AU
B. Thuringiensis ToxN: AAA^AA
L. lactis ToxN (AbiQ): AA^AA
[Eubacterium] rectale ToxN: GA^AAG

### Example 3 - Endoribonuclease activity - determining optimal enzyme concentration

The experiment was performed in order to verify that the ToxN (NCBI Acc. No.: WP_059274511) of use according to the invention exhibit endoribonuclease activity on a single-stranded RNA substrate.

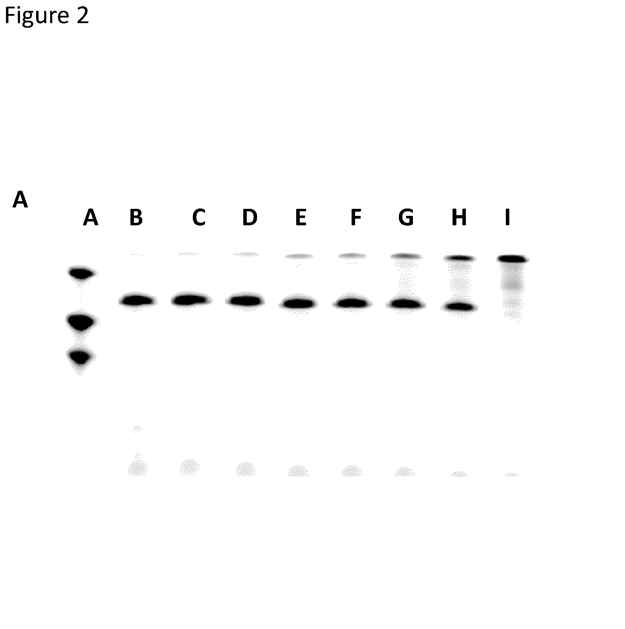

Digestion of a 50 nucleotide RNA single strand oligo with ToxN. The recognition site GAAAU is in the centre of the oligo and results in a product of 26 nucleotides. Results from analysis on a Polyacrylamid/Urea gel are shown in figure 2.
Sequence of the 50 nucleotide RNA single strand oligo with cleavage site for ToxN underlined:
   5'-CGCAAUUGCCGCAUUACAAGGGAAAUAACUUCGUACGUUGUUGUUAGCAU/3'-FAM
Assay conditions (25 µl) :
   0 nM to 120 nM ToxN enzyme
   50 mM Tris-HCl pH 7.5
   50 mM NaCl
   1mM DTT
   0.125 µM RNA oligo
   1 h at 15 °C

### Example 4 - Activity profiling of ToxN endoribonucleases: concentration of monovalent salt and pH

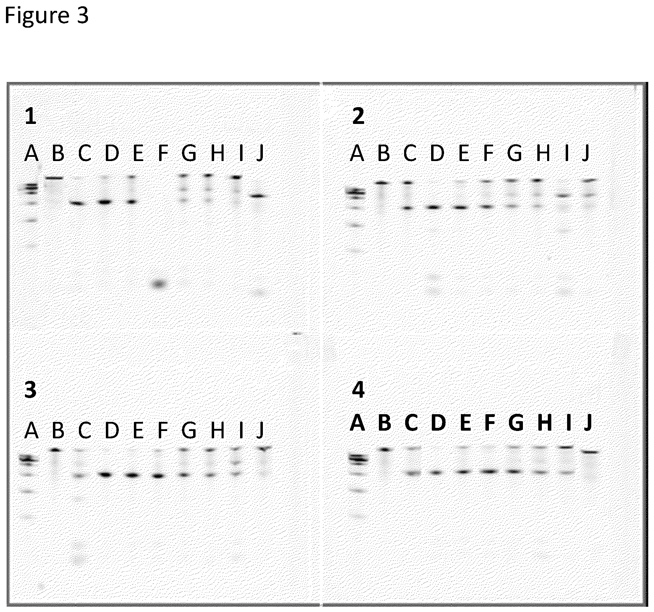

Digestion of a 50 nucleotide RNA single strand oligo with ToxN. The recognition site GAAAU is in the centre of the oligo and results in a product of 26 nucleotides (the sequence of the oligo is shown in Example 3). The results are shown in figure 3 and demonstrates that the ToxN enzyme are able to cleave single stranded RNA at pH ranging from 7.0 to 9.0. The results further demonstrate that the specificity of the enzyme is dependent on monovalent ion concentration and is optimal at NaCl concentrations of 50 mM and lower. Above 50 mM the cleavage of the RNA is less specific and the enzyme digests RNA at secondary closely related sequence recognition sites.

### Assay conditions

125 nM RNA oligo
40 nM ToxN
50 mM Tris-HCl pH 7.0 to pH 9.0
NaCl 0 mM to 175 mM
1 h at 15 °C

Results from analysis on a Polyacrylamide/Urea gel

### Example 5 - Activity profiling of ToxN endoribonucleases: concentration of divalent salt

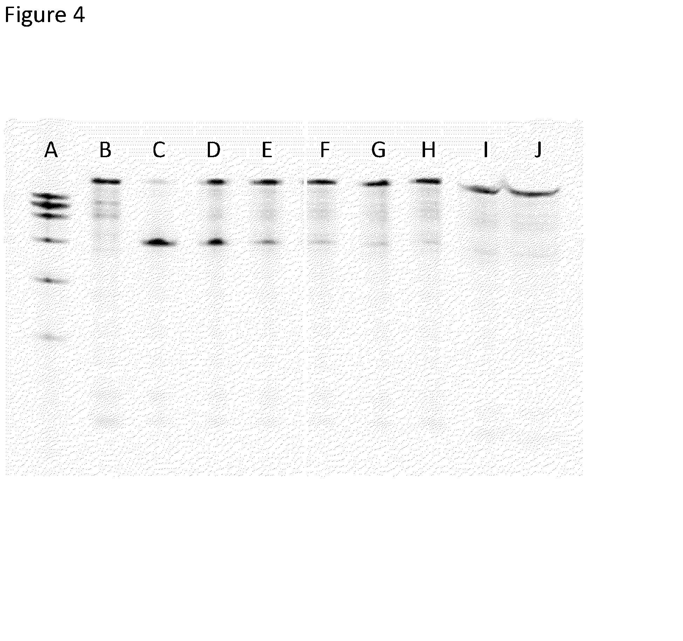

Digestion of a 50 nucleotide RNA single strand oligo with ToxN. The recognition site GAAAU is in the centre of the oligo and results in a product of 26 nucleotides (the sequence of the oligo is shown in Example 3). The results are shown in figure 4. ToxN enzyme activity is extensively inhibited by concentrations of MgCl₂ above 1mM (lane E - lane J).

| **A** | **B** | **C** | **D** | **E** | **F** | **G** | **H** | **I** | **J** |
|---|---|---|---|---|---|---|---|---|---|
| MW DNA ladder | w/o ToxN enzyme | ToxN 0mM MgCl₂ | ToxN 1mM MgCl₂ | ToxN 2mM MgCl₂ | ToxN 3mM MgCl₂ | ToxN 4mM MgCl₂ | ToxN 5mM MgCl₂ | ToxN 6mM MgCl₂ | ToxN 7mM MgCl₂ |

### Assay conditions

50 mM TrisHCl pH 8.0
25 mM NaCl
125 nM RNA oligo
40 nM ToxN
1 h at 15 °C

### Example 6 - Activity profiling of ToxN endoribonucleases: concentration of divalent salt, EDTA and DTT

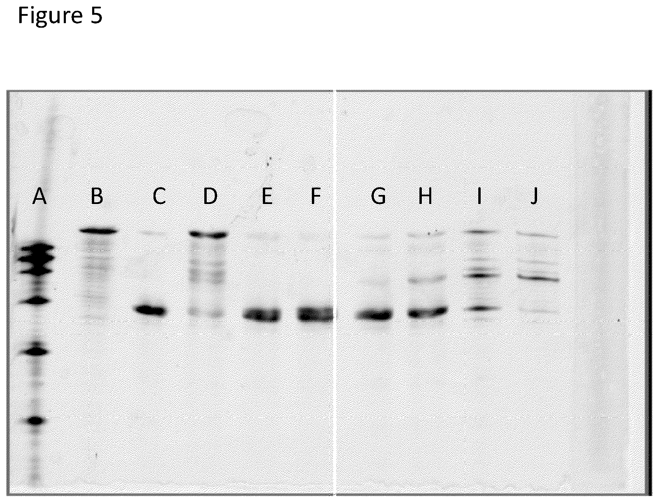

Digestion of a 50 nucleotide RNA single strand oligo with ToxN recognition sequence. The recognition site GAAAU is in the centre of the oligo and results in a product of 26 nucleotides (the sequence of the oligo is shown in Example 3). The results from the experiment is shown in figure 5 which demonstrates that ToxN endoribonuclease activity is regained by the addition of EDTA, a divalent cation scavenger (lanes G, H).

| **A** | **B** | **C** | **D** | **E** | **F** | **G** | **H** | **I** | **J** |
|---|---|---|---|---|---|---|---|---|---|
| MW DNA ladder | w/o ToxN enzyme | ToxN | ToxN 5mM MgCl₂ | ToxN 5mM EDTA | ToxN 5mM DTT | ToxN 5mM MgCl₂ | ToxN 5mM MgCl₂ | ToxN 5mM MgCl₂ | ToxN 5mM MgCl₂ |
| | | | | | | 5mM EDTA | 10mM EDTA | 5mM EDTA | 10mM EDTA |
| | | | | | | | | 5mM DTT | 5mM DTT |

### Assay conditions

50 mM TrisHCl pH 8.0
25 mM NaCl
125 nM RNA oligo
40 nM ToxN
1 h at 15 °C

### Example 7 - Activity profiling of ToxN endoribonucleases: incubation time at 15°C

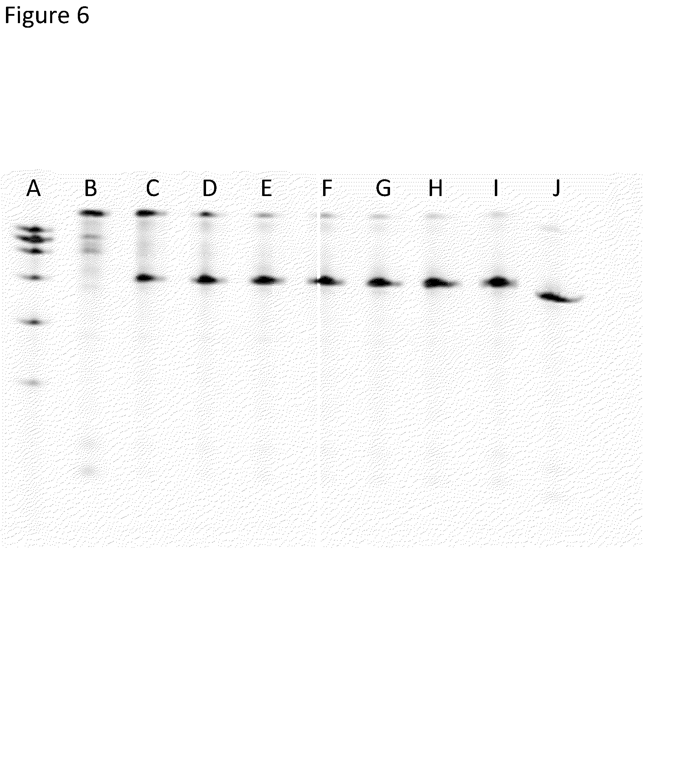

Digestion of a 50 nucleotide RNA single strand oligo with ToxN at increasing incubation time. The recognition site GAAAU is in the centre of the oligo and results in a product of 26 nucleotides (the sequence of the oligo is shown in Example 3). The results are shown in figure 6 and demonstrates that the enzyme is efficient. 50% of the RNA oligos are cleaved after 5 min at an optimal assay temperature of 15°C (lane C).

| **A** | **B** | **C** | **D** | **E** | **F** | **G** | **H** | **I** | **J** |
|---|---|---|---|---|---|---|---|---|---|
| MW DNA ladder | w/o ToxN enzyme | ToxN 5min. | ToxN 10min. | ToxN 15min. | ToxN 20min. | ToxN 30min. | ToxN 40min. | ToxN 50min. | ToxN 60min. |

### Assay conditions

50 mM Tris-HCl pH 8.0
25 mM NaCl
125 nM RNA oligo
40 nM ToxN
15 °C

### Example 8 - Activity profiling of ToxN endoribonucleases: incubation temperature

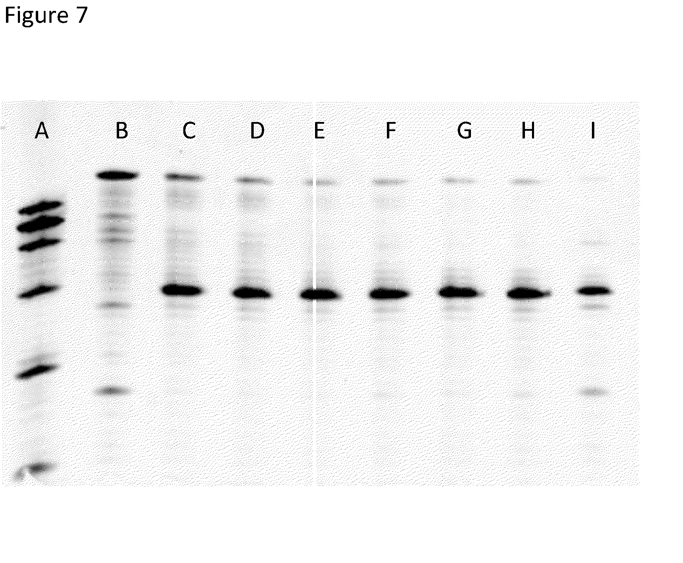

Digestion of a 50 nucleotide RNA single strand oligo with ToxN. The recognition site GAAAU is in the centre of the oligo and results in a product of 26 nucleotides (the sequence of the oligo is shown in Example 3). The aim of the study was to profile the enzyme activity at different incubation temperatures. The results are shown in figure 7.

| **A** | **B** | **C** | **D** | **E** | **F** | **G** | **H** | **I** |
|---|---|---|---|---|---|---|---|---|
| MW DNA ladder | w/o ToxN enzyme | ToxN 6 °C | ToxN 7 °C | ToxN 10 °C | ToxN 13 °C | ToxN 17 °C | ToxN 22 °C | ToxN 27 °C |

50 mM TrisHCl pH 8.0
25 mM NaCl
125 nM RNA oligo
40 nM ToxN
30 min at the specified temperatures

### Example 9 - Activity profiling of ToxN endoribonucleases: incubation temperature

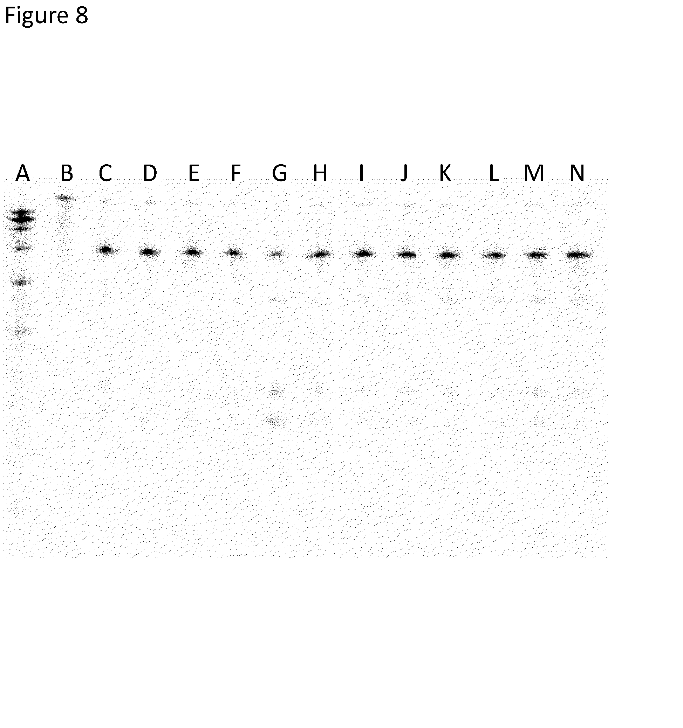

Digestion of a 50 nucleotide RNA single strand oligo with ToxN. The recognition site GAAAU is in the centre of the oligo and results in a product of 26 nucleotides (the sequence of the oligo is shown in Example 3). The aim of the study was to profile the enzyme activity at different incubation temperatures. The results are shown in figure 8 and demonstrates that the enzyme specificity decreases at temperatures above 30°C.

| **A** | **B** | **C** | **D** | **E** | **F** | **G** | **H** | **I** |
|---|---|---|---|---|---|---|---|---|
| MW DNA ladder | w/o ToxN enzyme | ToxN 15 °C | ToxN 16 °C | ToxN 17 °C | ToxN 20 °C | ToxN 23 °C | ToxN 27 °C | ToxN 32 °C |

| **J** | **K** | **L** | **M** | **N** |
|---|---|---|---|---|
| ToxN 37 °C | ToxN 40 °C | ToxN 43 °C | ToxN 44 °C | ToxN 45 °C |

### Assay conditions

50 mM TrisHCl pH 8.0
25 mM NaCl
125 nM RNA oligo
40 nM ToxN
30 min at the specified temperatures

### Sequence listing

| **SEQ ID NO** | **NCBI or UniProt Acc. No.** | **Description** |
|---|---|---|
| 1 | PDB: 7D8O_A | Amino acid sequence type III toxin-antitoxin system ToxN/AbiQ family toxin [Escherichia] |
| | MAKFFTISSSYIKYLKDFDDKVPNSEDPTYNNPK AFIGIVLEIEGHKYLAPLTSPKAWHANVKESSPA FFKLHENGVPDNQLGLINLKFMIPIIEAEVSLLD LDSMPDTPYKRMLYKQLQFIRVNEDKISEKSKLL RNLALQGRMQGTCDFAVLEEKYQHFGKKPEDM EIDDLESR | |
| 2 | PDB: 7D8O_A | Amino acid sequence type III toxin-antitoxin system ToxN/AbiQ family toxin [Escherichia] incl. N-terminal His-tag |
| | MNHKVHHHHHHMAKFFTISSSYIKYLKDFDDKV PNSEDPTYNNPKAFIGIVLEIEGHKYLAPLTSP KAWHANVKESSPAFFKLHENGVPDNQLGLINLK FMIPIIEAEVSLLDLDSMPDTPYKRMLYKQLQF IRVNEDKISEKSKLLRNLALQGRMQGTCDFA VLEEKYQHFGKKPEDMEIDDLESR | |
| 3 | ATGAAATTTTTCACAATATCAAGCAGTTACATA AAATACCTGAAGGACTTTGATGACAAAGTTCCCA ATAGCGAAGATCCTACATACAACAATCCTAAGGCT TTCATTGGCATAGTACTAGAGATTGAAGGACATA AATATTTAGCACCTTTAACATCGCCAAAAGCATGG CATGCTAATGTAAAAGAGTCATCACCCGCCTTCTT CAAACTTCATGAAAACGGTGTCCCTGATAATCAGC TTGGATTGATTAATCTGAAATTTATGATTCCAATAA TCGAAGCTGAAGTGTCTTTACTTGATCTGGATAGC | DNA sequence encoding ToxN with amino acid sequence with SEQ ID No. 1 and DNA sequence |
| | ATGCCTGATACGCCTTACAAAAGAATGCTATATAA ACAGTTACAATTTATTCGTGTAAATGAAGATAAAA TATCTGAAAAATCAAAACTATTAAGAAACCTTGCC TTGCAAGGCAGAATGCAAGGAACATGCGACTTTGCC GTGTTAGAAGAAAAATATCAACATTTCGGTAAAAAA CCTGAAGACATGGAAATAGACGATTAA | |
| 4 | AGGTGATTTGCTACCTTTAAGTGCAGCTAGAA ATTTAGGTGATTTGCTACCTTTAAGTGCAGCTAG AAATTTAGGTGATTTGCTACCTTTAAGTGCAGCTA GAAATTCAGGTGATTTGCTACCTTTAAGTGCAGCT AGAAATTTAGGTCATTTACTACCTTAAAGT | DNA sequence ToxI antitoxin repeats |
| 5 | AUUCAGGUGAUUUGCUACCUUUAAGUGCAGCUAGAA | RNA molecule ToxI antitoxin single strand repeat of ToxIN complex |
| 6 | B8X8Z0 | Amino acid sequence type III toxin-antitoxin system ToxN/AbiQ family toxin [Pectobacterium atrosepticum] |
| | MKFYTISSKYIEYLKEFDDKVPNSEDPTYQNPKA FIGIVLEIQGHKYLAPLTSPKKWHNNVKESSLSC FKLHENGVPENQLGLINLKFMIPIIEAEVSLLDL GNMPNTPYKRMLYKQLQFIRANSDKIASKSDTLRN LVLQGKMQGTCNFSLLEEKYRDFGKEAEDTEEGE | |
| 7 | Q3YN09 | Amino acid sequence type III toxin-antitoxin system ToxN/AbiQ family toxin [Bacillus cereus group] |
| | MTNKDNPKFHTISTEYIDYLREADSKVPFNKDEQHSRP YVGVLEKINGHDYFVPLTSRNDKNFNSQVSVKLFDND EKRIGVLLVNNMIPVPEKECKEIDIAEKTAADPQYGN LMLKQYLFLKENMDRVTNKVEKVYKDVTVQGKPSHKQ KFLKGVCCDFPKLEEKCQEYKERDQAKERDKARRIAY MRQMGRER | |
| 8 | Q9ZJ19.1 | Amino acid sequence sequence type III toxin-antitoxin system ToxN/AbiQ family toxin |
| | MSSFFYKEILRMTLRFFTVTDEYIAYLRKFESKV HYQYENNASTYVGVVLKKNDFNYFIPLSSYKKGN PEKDKAMKKRSRIVTRLFEIGNINNPLGYLLHHNM IPVPDSELIPLPLDLKKPKHKMMQKQLIYMKSISE KIENKSEVVYRKAAHEKDGYYLKFSCDFKLLEAKAT LYSKKSTFQ | |
| | | [Lactococcus lactis subsp. lactis] |
| 9 | CBK89509.1 | CptIN subfamily of the type III Toxin Antitoxin Systems [Eubacterium] rectale] |
| | MIRNGFYIIKDRFFSDMSDPYLKGNKKQNRPHYYCFE DSNYNGIYWMIPLSSRIDKYKKIVSKRTGKGRNCDIIH IVKLDDSHESAFLIQDMFPISDKYIEREYTIAGNHLRL TSEHAAKEIEQKARKVLGMLKRGIKFTPTQPDIQKIYE RLQQDLPATHL | |
| 10 | CGCAAUUGCCGCAUUACAAGGGAAAUAACUUCGUA CGUUGUUGUUAGCAU | RNA molecule comprising cleavage site for ToxN comprises the amino acid sequence SEQ ID No. 1 |
| 11 | AGGTCATATGGCGAAATTTTTCACAATATCA | Forward primer for cloning ToxN SEQ ID NO. 3 |
| 12 | GGAAATAGACGATCTCGAGTCTAGAGCAT | Revers primer for cloning ToxN SEQ ID NO. 3 |

## Claims

1. A method of cleaving single stranded RNA molecules in a sample, wherein the method comprises the steps:
a. providing a sample comprising at least one single stranded RNA molecule comprising a cleavage site for a ToxN endoribonuclease, wherein said single stranded RNA molecule is a concatemer comprising multiple copies of precursors of either mRNA, siRNA, circular RNA, microRNA or ribozyme and wherein the concatemeric RNA molecule comprises a cleavage site for a ToxN endoribonuclease between each copy of precursor of mRNA, siRNA, circular RNA, microRNA or ribozyme; and
b. contacting a ToxN endoribonuclease or an enzymatically active fragment thereof with the at least one RNA molecule in said sample under conditions which permits cleavage of at least a portion said RNA molecule present in the sample and wherein concentration of a monovalent salt in the sample is ≤150 mM, and wherein the monovalent salt is an alkali metal salt.

2. A method for determining 5'-capping efficiency of a RNA molecule wherein the method comprises the steps:
a. providing a sample comprising at least one single stranded mRNA molecule comprising a cleavage site for a ToxN endoribonuclease in the 5'UTR of said mRNA;
b. contacting said sample from step a) with a ToxN endoribonuclease under conditions that permits cleavage of at least a portion of said single stranded RNA molecules to produce at least one 5' terminal RNA fragment and at least one 3' terminal RNA fragment, wherein concentration of a monovalent salt in the sample is ≤150 mM, and wherein the monovalent salt is an alkali metal salt; and
c. separating and detecting the RNA fragments from step b) and determine the presence of a 5'-capping modification at the 5'end of said 5' terminal RNA fragments.

3. A method of RNA fingerprinting, wherein the method comprises the steps:
a. providing a sample comprising a at least one single stranded RNA molecule with unknown sequence;
b. contacting said sample from step a) with a ToxN endoribonuclease under conditions that permit cleavage of at least a portion of said RNA molecules thereby obtaining a plurality of RNA fragments, wherein concentration of a monovalent salt in the sample is ≤150 mM, and wherein the monovalent salt is preferably an alkali metal salt; and
c. separation and detection of the fragmented RNA molecule from step b, thereby obtaining a fingerprint of said RNA molecule with unknown sequence and compare the obtained fingerprint with fingerprints from RNA molecules with known sequence.

4. The method according to claim 2 or 3, wherein the separation and detection step is selected from gel electrophoresis, capillary gel-electrophoresis (CGE), PAGE, high pressure liquid chromatography (HPLC), mass spectrometry (MS) or LC-MS, IP RP HPLC and LC-UV.

5. The method according to claim 3, wherein the RNA molecule is selected from mRNA, RNA virus, an immunogenic RNA molecule, viroid, long non-coding RNA and ribozyme.

6. The method according to any one of claims 1 to 5, wherein the sample is essentially without Mg²⁺ or Mn²⁺.

7. The method according to any one of claims 1 to 5, wherein a concentration of divalent metal cations in the sample is ≤ 3mM, wherein the divalent metal cations are Mg²⁺ or Mn²⁺.

8. The method according to any one of claims 1 to 6, wherein the sample comprises a concentration of a divalent ion chelating agent of ≤ 10mM.

9. The method according to any one of claims 1 to 6, wherein the isolated ToxN endoribonuclease or an enzymatically fragment thereof comprises amino acid sequence of SEQ ID No.1 or comprising an amino acid sequence which is at least 70% identical to SEQ ID No.1.

## Patentansprüche

1. Verfahren zum Spalten von einzelsträngigen RNA-Molekülen in einer Probe, wobei das Verfahren folgende Schritte umfasst:
a. Bereitstellen einer Probe, die mindestens ein einzelsträngiges RNA-Molekül umfasst, das eine Spaltstelle für eine ToxN-Endoribonuklease umfasst, wobei das einzelsträngige RNA-Molekül ein Concatemer ist, das mehrere Kopien von Vorläufern entweder von mRNA, siRNA, zirkulärer RNA, microRNA oder Ribozym umfasst, und wobei das concatemere RNA-Molekül eine Spaltstelle für eine ToxN-Endoribonuklease zwischen jeder Kopie des Vorläufers von mRNA, siRNA, zirkulärer RNA, microRNA oder Ribozym umfasst, und
b. Inkontaktbringen einer ToxN-Endoribonuclease oder eines enzymatisch aktiven Fragments davon mit dem mindestens einem RNA-Molekül in der Probe unter Bedingungen, die eine Spaltung mindestens eines Teils des in der Probe vorhandenen RNA-Moleküls ermöglichen, und wobei die Konzentration eines monovalenten Salzes in der Probe ≤ 150 mM beträgt und wobei das monovalente Salz ein Alkalimetallsalz ist.

2. Verfahren zum Bestimmen der 5'-Capping-Effizienz eines RNA-Moleküls, wobei das Verfahren folgende Schritte umfasst:
a. Bereitstellen einer Probe, die mindestens ein einzelsträngiges mRNA-Molekül umfasst, das eine Spaltstelle für eine ToxN-Endoribonuklease in der 5'-UTR der mRNA umfasst,
b. Inkontaktbringen der Probe aus Schritt a) mit einer ToxN-Endoribonuklease unter Bedingungen, die eine Spaltung von mindestens einem Teil der einzelsträngigen RNA-Moleküle ermöglichen, um mindestens ein 5'-terminales RNA-Fragment und mindestens ein 3'-terminales RNA-Fragment zu erzeugen, wobei die Konzentration eines monovalenten Salzes in der Probe ≤ 150 mM beträgt und wobei das monovalente Salz ein Alkalimetallsalz ist, und
c. Auftrennen und Nachweisen der RNA-Fragmente aus Schritt b) und Bestimmen des Vorhandenseins einer 5'-Capping-Modifikation am 5'-Ende der 5'-terminalen RNA-Fragmente.

3. Verfahren zum RNA-Fingerabdruck, wobei das Verfahren folgende Schritte umfasst:
a. Bereitstellen einer Probe, die mindestens ein einzelsträngiges RNA-Molekül mit unbekannter Sequenz umfasst;
b. Inkontaktbringen der Probe aus Schritt a) mit einer ToxN-Endoribonuklease unter Bedingungen, die eine Spaltung von mindestens einem Teil der RNA-Moleküle ermöglichen, wodurch eine Vielzahl von RNA-Fragmenten erhalten wird, wobei die Konzentration eines monovalenten Salzes in der Probe ≤ 150 mM beträgt und wobei das monovalente Salz vorzugsweise ein Alkalimetallsalz ist, und
c. Auftrennung und Nachweis des fragmentierten RNA-Moleküls aus Schritt b, wodurch ein Fingerabdruck des RNA-Moleküls mit unbekannter Sequenz erhalten wird, und Vergleichen des erhaltene Fingerabdrucks mit Fingerabdrücken von RNA-Molekülen mit bekannter Sequenz.

4. Verfahren gemäß Anspruch 2 oder 3, wobei der Trennungs- und Nachweisschritt ausgewählt ist aus Gelelektrophorese, Kapillar-Gelelektrophorese (CGE), PAGE, Hochdruckflüssigkeitschromatographie (HPLC), Massenspektrometrie (MS) oder LC-MS, IP RP HPLC und LC-UV.

5. Verfahren gemäß Anspruch 3, wobei das RNA-Molekül ausgewählt ist aus mRNA, RNA-Virus, einem immunogenen RNA-Molekül, Viroid, langer nichtkodierender RNA und Ribozym.

6. Verfahren gemäß einem der Ansprüche 1 bis 5, wobei die Probe im Wesentlichen ohne Mg²⁺ oder Mn²⁺ ist.

7. Verfahren gemäß einem der Ansprüche 1 bis 5, wobei eine Konzentration von zweiwertigen Metallkationen in der Probe ≤ 3 mM beträgt, wobei die zweiwertigen Metallkationen Mg²⁺ oder Mn²⁺ sind.

8. Verfahren gemäß einem der Ansprüche 1 bis 6, wobei die Probe eine Konzentration eines Chelatbildners für zweiwertige Ionen von ≤ 10 mM umfasst.

9. Verfahren gemäß einem der Ansprüche 1 bis 6, wobei die isolierte ToxN-Endoribonuklease oder ein enzymatisches Fragment davon eine Aminosäuresequenz von SEQ ID NO. 1 umfasst oder eine Aminosäuresequenz umfasst, die zu mindestens 70 % mit SEQ ID NO. 1 identisch ist.

## Revendications

1. Procédé de clivage de molécules d'ARN simple brin dans un échantillon, dans lequel le procédé comprend les étapes de :
a. fourniture d'un échantillon comprenant au moins une molécule d'ARN simple brin comprenant un site de clivage pour une endoribonucléase ToxN, dans lequel ladite molécule d'ARN simple brin est un concatémère comprenant de multiples copies de précurseurs d'ARNm, d'ARNsi, d'ARN circulaire, de microARN ou de ribozyme et dans lequel la molécule d'ARN concatémérique comprend un site de clivage pour une endoribonucléase ToxN entre chaque copie de précurseur d'ARNm, d'ARNsi, d'ARN circulaire, de microARN ou de ribozyme ; et
b. mise en contact d'une endoribonucléase ToxN ou d'un fragment enzymatiquement actif de celle-ci avec l'au moins une molécule d'ARN dans ledit échantillon dans des conditions permettant le clivage d'au moins une partie de ladite molécule d'ARN présente dans l'échantillon, et dans lequel la concentration d'un sel monovalent dans l'échantillon est ≤150 mM, et dans lequel le sel monovalent est un sel de métal alcalin.

2. Procédé permettant la détermination de l'efficacité de coiffage en 5' d'une molécule d'ARN, dans lequel le procédé comprend les étapes de :
a. fourniture d'un échantillon comprenant au moins une molécule d'ARNm simple brin comprenant un site de clivage pour une endoribonucléase ToxN dans l'UTR 5' dudit ARNm ;
b. mise en contact dudit échantillon de l'étape a) avec une endoribonucléase ToxN dans des conditions permettant le clivage d'au moins une partie desdites molécules d'ARN simple brin pour produire au moins un fragment d'ARN terminal 5' et au moins un fragment d'ARN terminal 3', dans lequel la concentration d'un sel monovalent dans l'échantillon est ≤150 mM, et dans lequel le sel monovalent est un sel de métal alcalin ; et
c. séparation et détection des fragments d'ARN de l'étape b) et détermination de la présence d'une modification de coiffage 5' à l'extrémité 5' desdits fragments d'ARN terminaux 5'.

3. Procédé d'empreinte d'ARN, dans lequel le procédé comprend les étapes de :
a. fourniture d'un échantillon comprenant au moins une molécule d'ARN simple brin de séquence inconnue ;
b. mise en contact dudit échantillon de l'étape a) avec une endoribonucléase ToxN dans des conditions permettant le clivage d'au moins une partie desdites molécules d'ARN, obtenant ainsi une pluralité de fragments d'ARN, dans lequel la concentration d'un sel monovalent dans l'échantillon est ≤150 mM, et dans lequel le sel monovalent est de préférence un sel de métal alcalin ; et
c. séparation et détection de la molécule d'ARN fragmentée de l'étape b, obtenant ainsi une empreinte de ladite molécule d'ARN de séquence inconnue et comparaison de l'empreinte obtenue avec des empreintes de molécules d'ARN de séquence connue.

4. Procédé selon la revendication 2 ou 3, dans lequel l'étape de séparation et de détection est choisie parmi l'électrophorèse sur gel, l'électrophorèse capillaire sur gel (CGE), la PAGE, la chromatographie liquide à haute pression (HPLC), la spectrométrie de masse (MS) ou la LC-MS, la HPLC IP RP et la LC-UV.

5. Procédé selon la revendication 3, dans lequel la molécule d'ARN est choisie parmi un ARNm, un virus à ARN, une molécule d'ARN immunogène, un viroïde, un ARN long non codant et un ribozyme.

6. Procédé selon l'une quelconque des revendications 1 à 5, dans lequel l'échantillon est essentiellement exempt de Mg²⁺ ou Mn²⁺.

7. Procédé selon l'une quelconque des revendications 1 à 5, dans lequel une concentration de cations métalliques divalents dans l'échantillon est ≤ 3 mM, dans lequel les cations métalliques divalents sont Mg²⁺ ou Mn²⁺.

8. Procédé selon l'une quelconque des revendications 1 à 6, dans lequel l'échantillon comprend une concentration d'un agent chélatant d'ions divalents ≤ 10 mM.

9. Procédé selon l'une quelconque des revendications 1 à 6, dans lequel l'endoribonucléase ToxN isolée ou un fragment enzymatique de celle-ci comprend une séquence d'acides aminés de SEQ ID N°1 ou comprenant une séquence d'acides aminés présentant au moins 70 % d'identité avec SEQ ID N°1.
